# EUROPEAN PATENT APPLICATION

(11) **EP 4 382 107 A1**
(43) Date of publication of application: **12.06.2024**
(21) Application number: 22853118.2
(22) Date of filing: 03.08.2022
(51) Int. Cl.: A61K 31/57, A61K 9/127, A61K 39/395, A61P 35/00, A61P 43/00

(54) **COMPANION DRUG COMPRISING ACTIVITY REGULATOR FOR T CELL AND/OR B CELL**

(30) Priority: 04.08.2021 JP 2021128012
(71) Applicant: Tokai University Educational System, Tokyo, 151-0063 (JP)
(72) Inventor: KAMETANI, Yoshie, Isehara-shi, Kanagawa 259-1193 (JP); MANABE, Yoshiyuki, Toyonaka-shi, Osaka 560-0022 (JP)
(74) Representative: Altmann Stößel Dick Patentanwälte PartG mbB
(86) International application number: PCT/JP2022/029854
(87) International publication number: WO 2023/013700

(57) **Abstract**

The present invention provides a novel activity regulator capable of regulating T cell and/or B cell activity. The activity regulator is a T cell and/or B cell activity regulator comprising progesterone or a derivative thereof as an active ingredient.

## Description

### Technical Field

The present invention relates to an agent for combination use which is for using a T cell and/or B cell activity regulator comprising progesterone or a derivative thereof as an active ingredient, in combination with an anti-human PD-L1 monoclonal antibody.

### Background Art

The placenta has properties of proliferation, infiltration, and angiogenesis, and in addition, a property of immunomodulation for allowing a mother body to accept an embryo, which none of the other organs has. Further, the trophoblast, which is the placental tissue on the embryo side, expresses a large number of cancer-related genes, and the metabolic system thereof is similar to that of cancer (Non Patent Literatures 1 to 3). On the other hand, a mother body maintains certain immune response to infectious diseases though she has the placenta (the tissue on the embryo side), and the trophoblast stops infiltration in the myometrium, and hence health of the mother body and the embryo can be maintained. Therefore, it is considered possible to stop cancer infiltration and release immunosuppression without causing immune deficiency by revealing mechanisms of pregnancy immunity and cancer immunity, and utilizing a difference therebetween, which may lead to a breakthrough treatment.

In a cancer-bearing state, in particular, in cancerous cachexia or the like, TNFα or IL-6 produced by inflammation occurring in relation to the progression of cancer produce glucocorticoid (GC) that is an anti-inflammatory steroid hormone, to suppress the whole acquired immune system (to reduce the ratio of a killer cell and antibody production). However, in pregnancy immunity, it is presumed that this suppression can be smaller, and in addition can be reversible (for example, only antibody production is reduced, or GVHD is recovered). It is also presumed that such a difference is caused due to a concentration gradient of progesterone (PG) that is a pregnancy-related steroid hormone produced in the human placenta in a large amount. Among pregnancy-related hormones, PG or a derivative thereof suppresses proliferation of tumor cells depending on its concentration (Non Patent Literatures 4 and 5), and on the other hand, regulates activation of an immunocompetent cell (Non Patent Literatures 6 to 13).

In recent years, owing to development of cancer molecular target drugs based on an immune checkpoint antibody, treatment of refractory solid cancer has largely progressed. For a long period of time, a large number of studies had been made based on the hypothesis that a cancer-bearing state is related to suppression of an immune system, and hence cancer might be eliminated by activating an immune system specifically to a cancer, but most of these studies could not have attained a remarkable effect excluding passive immunity by an antibody against HER2 or the like. However, antibodies against an immune checkpoint molecule, represented by ipilimumab (CTLA-4 antibody), nivolumab (PD-1), and atezolizumab (PD-L1), exhibited a breakthrough antitumor effect, and thus cancer treatment has been newly progressed (Non Patent Literatures 14 and 15). This result indicates that there is a strong connection between cancer and abnormality of an immune system, and that release of suppression of an immune system plays a critical role in an anticancer effect. The effect is, however, limited, and a method designated as combination checkpoint blockage (CCB) using both of an antibody against CTLA-4 and an antibody against PD-1 has been tried in recent years. In this treatment, however, immune-related adverse events (IRAEs) have been observed as stronger side effects, which is a serious problem of this treatment. Regarding risk of these side effects, lymphocytes of various patients have been analyzed, but a clear guideline has not been established.

### Citation List

### Non Patent Literature

Non Patent Literature 1: Hayakawa S. No cancer in cancers: evolutionary trade-off between successful viviparity and tumor escape from the adaptive immune system. Med Hypotheses (2006) 66:888-97.
Non Patent Literature 2: Racicot K, JY K, Aldo P, Silasi M, Mor G. Understanding the complexity of the immune system during pregnancy. Am J Reprod Immunol (2014) 72:107-16. Non Patent Literature 3: Haig D. Maternal-fetal conflict, genomic imprinting and mammalian vulnerabilities to cancer. Philos Trans R Soc Lond B Biol Sci (2015) 370:20140178.
Non Patent Literature 4: Yahya S, Abdelhamid A, Abd-Elhalim M, Elsayed G, Eskander E. The effect of newly synthesized progesterone derivatives on apoptotic and angiogenic pathway in MCF-7 breast cancer cells. Steriods (2017) 126:15-23.
Non Patent Literature 5: You S, Zuo L, Li W. Optimizing the time of Doxil injection to increase the drug retention in transplanted murine mammary tumors. Int J Nanomedicine (2010) 5:221-9.
Non Patent Literature 6: Ndiaye K, Poole D, Walusimbi S, Cannon M, Toyokawa K, Maalouf S, et al. Progesterone effects on lymphocytes may be mediated by membrane progesterone receptors. J Reprod Immunol (2012) 95:15-26.
Non Patent Literature 7: Shah N, Imami and N, Johnson M. Progesterone Modulation of Pregnancy-related immune responses Front in Immunol (2018) 9:1293.
Non Patent Literature 8: Hierweger A, Engler J, Friese M, Reichardt H, Lydon J, DeMayo F, et al. Progesterone modulates the T cell response via glucocorticoid receptor-dependent pathways. Am J Reprod Immunol (2019) 81:e13084.
Non Patent Literature 9: Yao Y, Li H, Ding J, Xia Y, Wang L. Progesterone impairs antigen-non-specific immune protection by CD8 T memory cells via interferon-γ gene hypermethylation. PLoS Pathog (2017) 13:e1006736.
Non Patent Literature 10: Monteiro C, Kasahara T, Sacramento P, Dias A, Leite S, Silva V, et al. Human pregnancy levels of estrogen and progesterone contribute to humoral immunity by activating TFH/B cell axis. Eur J Immunol (2020) ahead of print.
Non Patent Literature 11: Piccinni M-P, Ciudizi M-C, Biagiotti R, Beloni L, Giannarini L, Sampognaro S, et al. Progesterone Favors the Development of Human T Helper Cells Producing Th2-Type Cytokines and Promotes Both 11-4 Production and Membrane CD30 Expression in Established Thl Cell Clones. J Immunol (1995) 155:128-33.
Non Patent Literature 12: Mauvais-Jarvis F, Klein S, Ellis R. Levin E. Estradiol, Progesterone, Immunomodulation, and COVID-19 Outcomes. Endocriology (2020) 161:1-8.
Non Patent Literature 13: Polikarpova A, Levin I, Sigai N, Zavarzin I, Morozovc I, Rubtsov P, et al. Immunomodulatory effects of progesterone and selective ligands of membrane progesterone receptors. Steroids (2019) 145:5-18.
Non Patent Literature 14: Patel S, Minn A. Combination Cancer Therapy with Immune Checkpoint Blockade: Mechanisms and Strategies. Immunity (2018) 48:417-33.
Non Patent Literature 15: He X, Xu C. Immune checkpoint signaling and cancer immunotherapy. Cell Res (2020) 30:660-9.
Non Patent Literature 16: Miyako H, Kametani Y, Katano I, Ito R, Tsuda B, Furukawa A, et al. Antitumor effect of new HER2 peptide vaccination based on B cell epitope. Anticancer Res (2011) 31(10):361-3368.
Non Patent Literature 17: Kametani Y, Shiina M, Katano I, Ito R, Ando K, Toyama K, e t al. Development of human-human hybridoma from anti-Her-2 peptide-producing B cells in immunized NOG mouse. Exp Hematol (2006) 34(9):1240-8.

### Summary of Invention

### Technical Problem

An object of the present invention is to provide a novel activity regulator capable of regulating T cell and/or B cell activity. Another object of the present invention is to provide an agent for combination use which is for using the T cell and/or B cell activity regulator in combination with an anti-human PD-L1 monoclonal antibody.

### Solution to Problem

In the present invention, progesterone or a derivative thereof is prescribed for a disease condition induced by activation of an immune system such as autoimmune disease-like side effects caused by an immune checkpoint inhibitor (ICI) to regulate immunity. The present inventors have presumed that progesterone can be used in a method for controlling IRAEs. The present inventors have first revealed a difference in the action for immunosuppression of progesterone and glucocorticoid, then revealed influence on an immune system of combined use of progesterone and atezolizumab, and further developed a conjugate of liposome encapsulating progesterone and atezolizumab to successfully perform immunoregulation more efficiently. The present invention has been accomplished based on these findings.

Specifically, the present invention provides the following inventions:
< 1 > An agent for combination use comprising, as an active ingredient, a T cell and/or B cell activity regulator comprising progesterone or a derivative thereof as an active ingredient, for use in combination treatment with an anti-human PD-L1 monoclonal antibody.
<2> The agent for combination use according to <1>, wherein the T cell and/or B cell activity regulator reversibly acts on a T cell and/or a B cell.
<3> The agent for combination use according to <1> or <2>, wherein the T cell and/or B cell activity regulator maintains expression of CD62L to localize a T cell in a lymph node.
<4> The agent for combination use according to any one of <1> to <3>, wherein the T cell and/or B cell activity regulator reduces expression of PD-1 in a T cell and/or a B cell.
<5> The agent for combination use according to any one of <1> to <4>, wherein the agent for combination use suppresses expression of PD-L1 in a T cell and/or a B cell.
<6> The agent for combination use according to any one of <1> to <5>, wherein the agent for combination use suppresses expression of PD-1 and PD-L1 in a T cell and/or a B cell.
<7> The agent for combination use according to any one of <1> to <6>, wherein the anti-human PD-L1 monoclonal antibody is a humanized anti-human PD-L1 monoclonal antibody.
<8> The agent for combination use according to any one of <1> to <7>, wherein the humanized anti-human PD-L1 monoclonal antibody is atezolizumab.
<9> A pharmaceutical composition comprising, as an active ingredient, a T cell and/or B cell activity regulator comprising progesterone or a derivative thereof as an active ingredient, and an anti-human PD-L1 monoclonal antibody.
< 10> The pharmaceutical composition according to <9>, wherein the T cell and/or B cell activity regulator reversibly acts on a T cell and/or a B cell.
<11> The pharmaceutical composition according to <9> or <10>, wherein the T cell and/or B cell activity regulator maintains expression of CD62L to localize a T cell in a lymph node.
< 12> The pharmaceutical composition according to any one of <9> to < 11 >, wherein the T cell and/or B cell activity regulator reduces expression of PD-1 in a T cell and/or a B cell.
< 13> The pharmaceutical composition according to any one of <9> to < 12>, wherein the pharmaceutical composition suppresses expression of PD-L1 in a T cell or a B cell.
< 14> The pharmaceutical composition according to any one of <9> to < 13>, wherein the pharmaceutical composition suppresses expression of PD-1 and PD-L1 in a T cell or a B cell.
< 15> The pharmaceutical composition according to any one of <9> to < 14>, wherein the anti-human PD-L1 monoclonal antibody is a humanized anti-human PD-L1 monoclonal antibody.
< 16> The pharmaceutical composition according to any one of <9> to < 15>, wherein the humanized anti-human PD-L1 monoclonal antibody is atezolizumab.
<17> An immunoliposome comprising a liposome comprising a T cell and/or B cell activity regulator comprising progesterone or a derivative thereof as an active ingredient, wherein an anti-human PD-L1 monoclonal antibody is bound to a membrane of the liposome.
<18> The immunoliposome according to <17>, wherein the T cell and/or B cell activity regulator reversibly acts on a T cell and/or a B cell.
<19> The immunoliposome according to <17> or <18>, wherein the T cell and/or B cell activity regulator maintains expression of CD62L to localize a T cell in a lymph node.
<20> The immunoliposome according to any one of <17> to <19>, wherein the T cell and/or B cell activity regulator reduces expression of PD-1 in a T cell and/or a B cell.
<21> The immunoliposome according to any one of <17> to <20>, wherein the immunoliposome suppresses expression of PD-L1 in a T cell and/or a B cell.
<22> The immunoliposome according to any one of <17> to <21>, wherein the immunoliposome suppresses expression of PD-1 and PD-L1 in a T cell and/or a B cell.
<23> The immunoliposome according to any one of <17> to <22>, wherein the anti-human PD-L1 monoclonal antibody is a humanized anti-human PD-L1 monoclonal antibody.
<24> The immunoliposome according to any one of <17> to <23>, wherein the humanized anti-human PD-L1 monoclonal antibody is atezolizumab.
<25> A pharmaceutical composition, comprising the immunoliposome according to any one of <17> to <24>.

The present specification encompasses the entire contents of Japanese Patent Application No. 2021-128012, based on which the application claims the benefit of priority.

### Advantageous Effects of Invention

With the agent for combination use of the present invention, T cell and/or B cell activation can be regulated so that efficient immunoregulation can be performed.

### Brief Description of Drawings

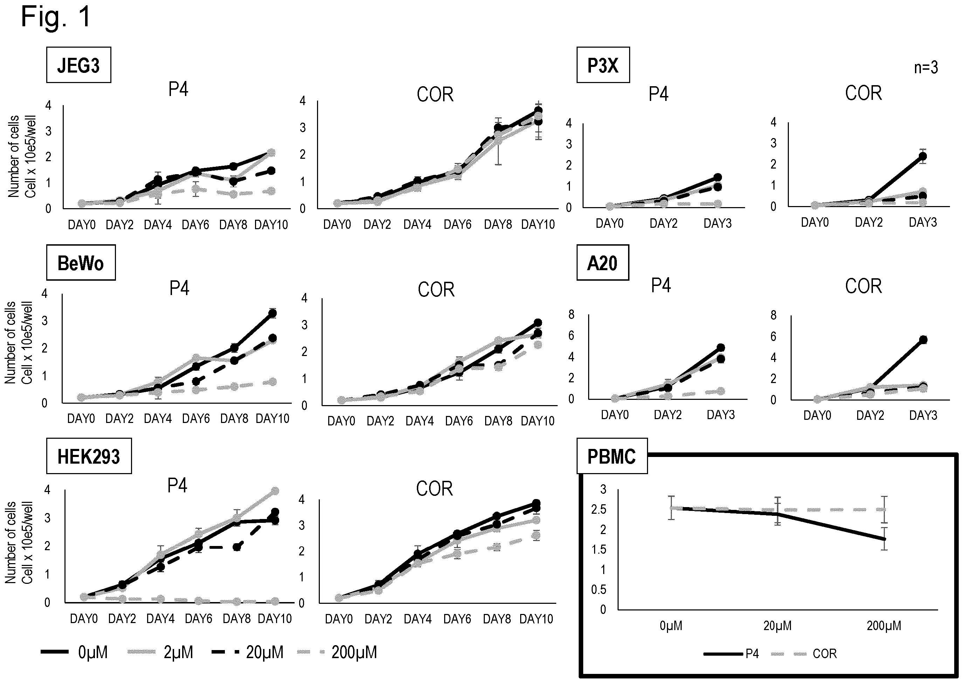

[Figure 1] Figure 1 illustrates the relationship between a P4/COR concentration and proliferating ability of various cell lines. Two panels on the left side illustrate results for JEG-3 and BeWo that are human choriocarcinoma cell lines, and HEK293 that is a kidney-derived cell line. Two panels on the right side illustrate results for P3X that is a mouse myeloma cell line, A20 that is a lymphoma cell line, and a human PBMC. P4 and COR were added thereto at concentrations of 0 to 200 µm, and the resultants were cultured for 10 days or 3 days. As for a PBMC, results of 3-day culture are illustrated.

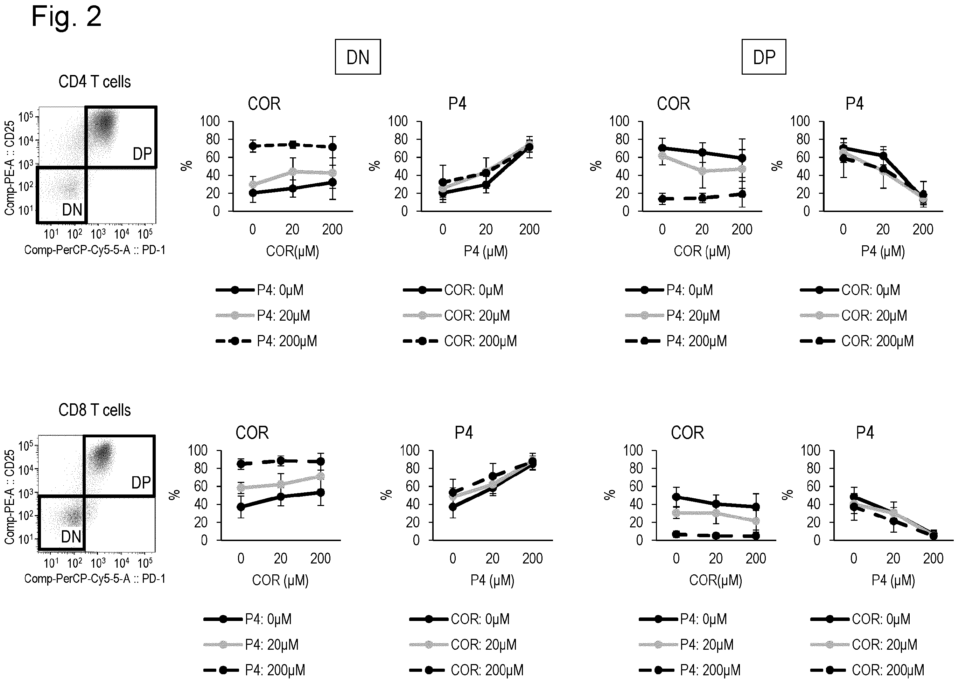

[Figure 2] Figure 2 illustrates the relationship (%) between a P4/COR concentration and PD-1 expression level on an activated T cell. Results of FCM analysis of PBMCs cultured for 3 days are illustrated. Two panels on the left side illustrate expression patterns of CD25 and PD-1 in a T cell obtained by FCM. CD4 T cells and CD8 T cells were gated respectively in upper panels and lower panels, and ratios of CD25 and PD-1 negative (double negative; DN) and positive (double positive; DP) fractions in CD4 T cells are illustrated. The ordinates of right graphs indicate a steroid concentration dependent ratio of each cell fraction. Two panels on the left side for CD4 or CD8 illustrate DN cells (P4 and COR), and two panels on the right side illustrate DP cells (similarly P4 and COR). As for P4, a COR or P4 concentration added is indicated on the abscissa to illustrate the relationships with the P4 and COR concentrations.

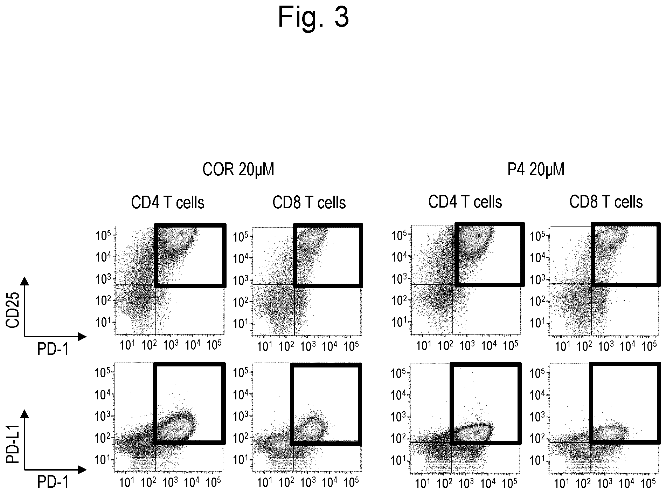

[Figure 3] Figure 3 illustrates CD25/PD-1/PD-L1 expression in activated T cells. Results of FCM analysis of PBMCs cultured for 3 days are illustrated. Upper panels illustrate development of PD-1 and CD25, and lower panels illustrate development of PD-1 and PD-L1. Two panels on the left side illustrate results of culture with 20 µM COR addition, and panels on the right side illustrate results of culture with 20 µM P4 addition, and analysis was further performed on a CD4 T cell and a CD8 T cell. A rectangle disposed in an upper right portion of each graph indicates a DP cell.

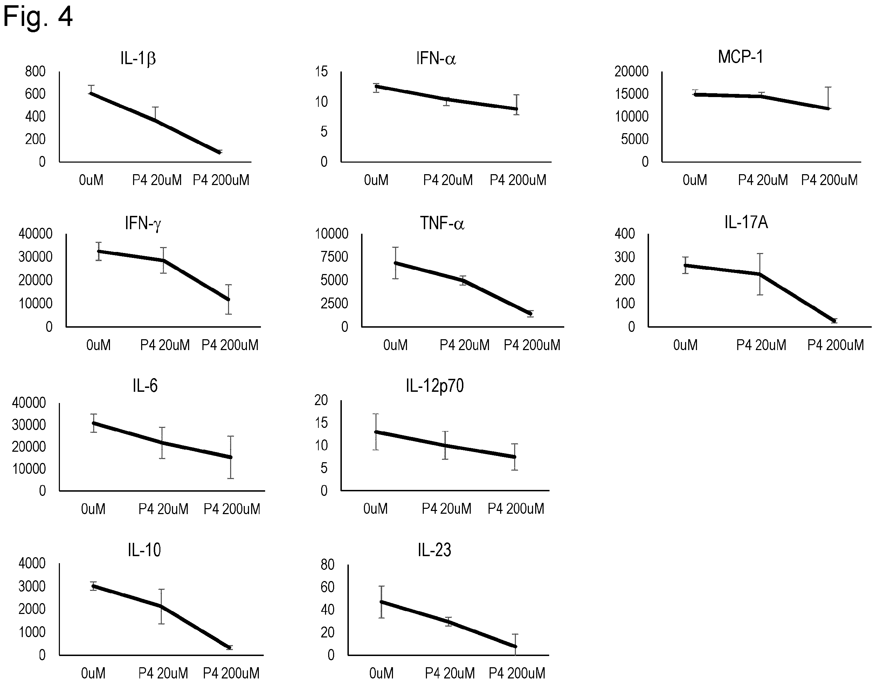

[Figure 4] Figure 4 illustrates cytokine production in PBMCs activated in the presence of P4. Results of FCM analysis of PBMCs cultured for 3 days are illustrated. The ordinate indicates a cytokine concentration (pg/mL) in culture supernatant obtained under conditions of 0 to 200 µM P4.

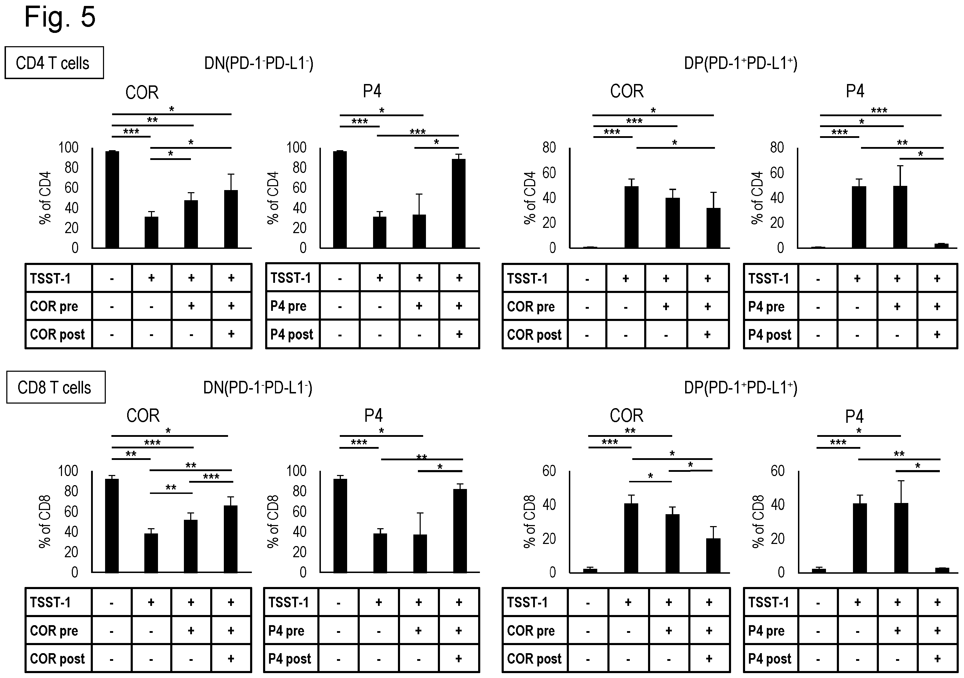

[Figure 5] Figure 5 illustrates the relationship between P4/COR pretreatment and a ratio of activated T cells. Specifically, it illustrates results of culturing PBMCs for 6 hours with 200 µM P4/COR addition, washing the resultant cells, culturing the resultant for 3 days with TSST-1 addition in the presence/absence of P4/COR, and analyzing the resultant by FCM. Upper panels illustrate a ratio of a PD-1/PD-L1 DN fraction and a ratio of a PD-1/PD-L1 DP fraction in CD4 T cells, and lower panels illustrate a ratio of a PD-1/PD-L1 DN fraction and a ratio of a PD-1/PD-L1 DP fraction in CD8 T cells. With respect to each fraction, the left panel illustrates results of culture with 200 µM COR addition, and the right panel illustrates those with 200 µM P4 addition.

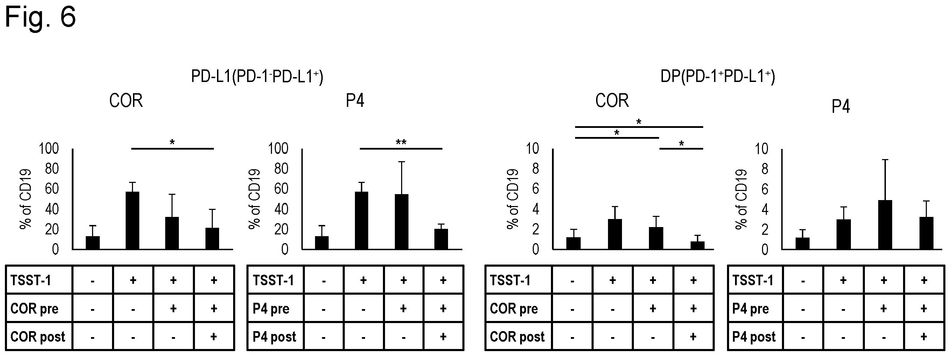

[Figure 6] Figure 6 illustrates the relationship between a P4/COR pretreatment and a ratio of a B cell. Specifically, it illustrates results of culturing PBMCs for 6 hours with 200 µM P4/COR addition, washing the resultant cells, culturing the resultant for 3 days with TSST-1 addition in the presence/absence of P4/COR, and analyzing the resultant by FCM. Two panels on the left side illustrate a PD-L1 SP cell, and two panels on the right side illustrate a PD-1/PD-L1 DP cell. With respect to each fraction, the left panel illustrates results of culture with 200 µM COR addition, and the right panel illustrates those with 200 µM P4 addition.

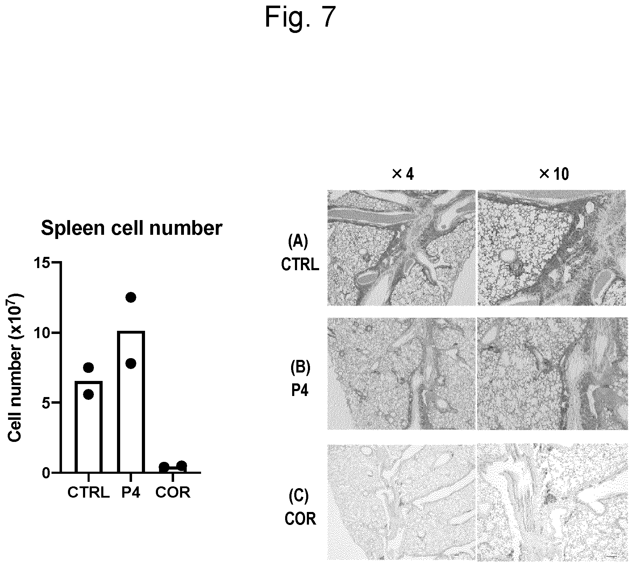

[Figure 7] Figure 7 illustrates engraftment ability of a human cell in a PBMC-NOG mouse having treated with P4/COR administration. Specifically, it illustrates the number of cells in the spleen and localization of human cells in the lung of a mouse to which human PBMCs were transplanted, and P4 or COR was continuously subcutaneously administered twice a week for 4 weeks. A graph on the left side illustrates averages of the number of the cells, and photographs on the right side illustrate results of immunohistochemical staining of a slice of the lung (including the bronchus) of the mouse with an anti-human CD45 antibody (leukocyte marker).

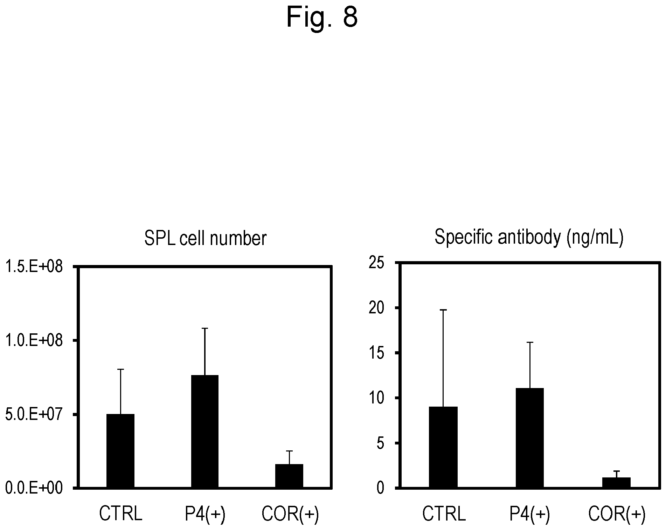

[Figure 8] Figure 8 illustrates engraftment ability of a human cell in a PBMC-NOG mouse to which PBMCs cultured with P4 or COR for a short time were transplanted. Specifically, it illustrates the number of spleen cells after 4 weeks (left panel) and a specific antibody titer in plasma after 4 weeks (right panel) in a mouse to which human PBMCs cultured with 200 µM P4/COR for 6 hours were transplanted, and which was immunized with CH401MAP every other week.

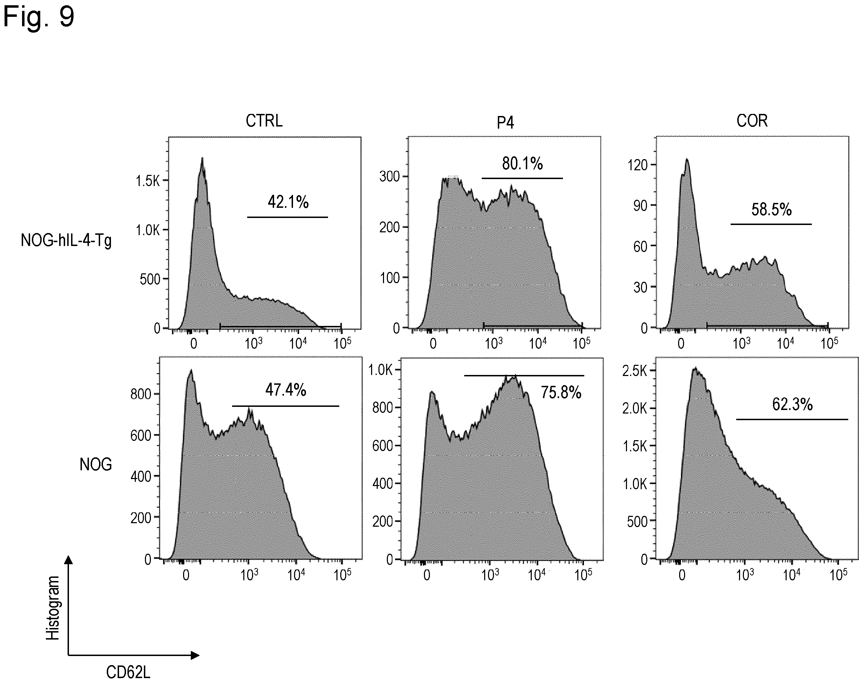

[Figure 9] Figure 9 illustrates CD62L expression in a spleen human lymphocyte in the mouse to which the PBMCs cultured with P4 or COR for a short time were transplanted as explained in Figure 7 or Figure 8. The expression of CD62L on a lymphocyte was confirmed by FCM. Upper panels illustrate CD62LMFI (CD45 gated) expressed in human CD45 positive cells in the spleen cells 4 weeks after transplantation to a humanized mouse using NOG-hIL-4-Tg, and lower panels illustrate CD62LMFI (CD45 gated) expressed in human CD45 positive cells in the spleen cells 4 weeks after transplantation to a humanized mouse using NOG. Each numerical value indicates a ratio of positive cells.

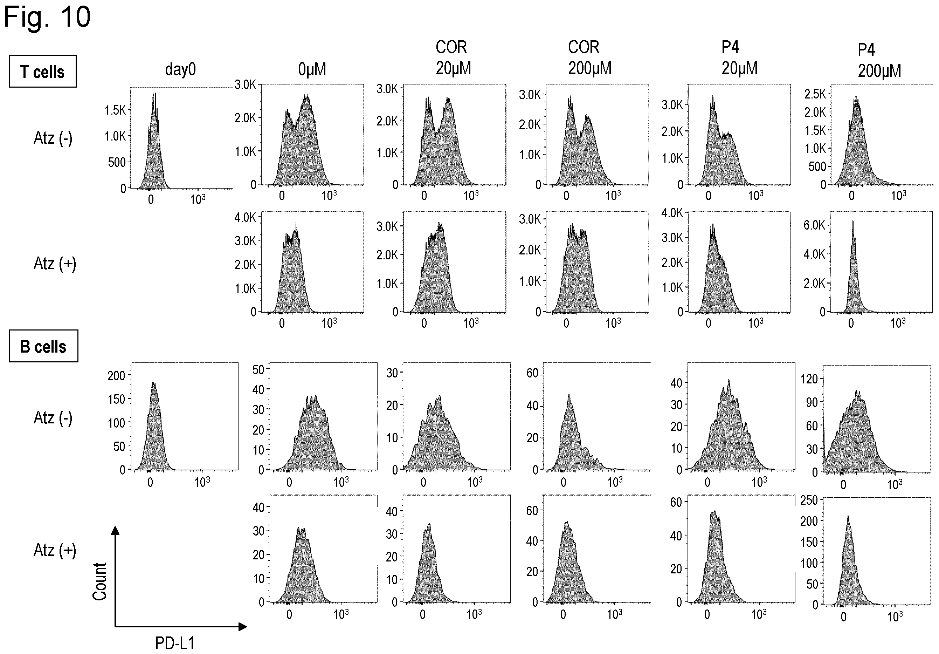

[Figure 10] Figure 10 illustrates the relationship between atezolizumab and PD-L1 expression in a lymphocyte. Human PBMCs were cultured for 3 days in a medium supplemented with 0 to 200 µM P4/COR and 100 µg/mL atezolizumab, and expression of PD-L1 on a lymphocyte was confirmed by FCM. Panels in upper two rows illustrate results obtained with a T cell (CD3 gated) and panels in lower two rows illustrate results obtained with a B cell (CD19 gated). Upper panels in each row illustrate results obtained without atezolizumab addition (Atz(-)), and lower panels illustrate results obtained with atezolizumab addition (Ats(+)). The ordinate indicates a count number, and the abscissa indicates expression level of PD-L1.

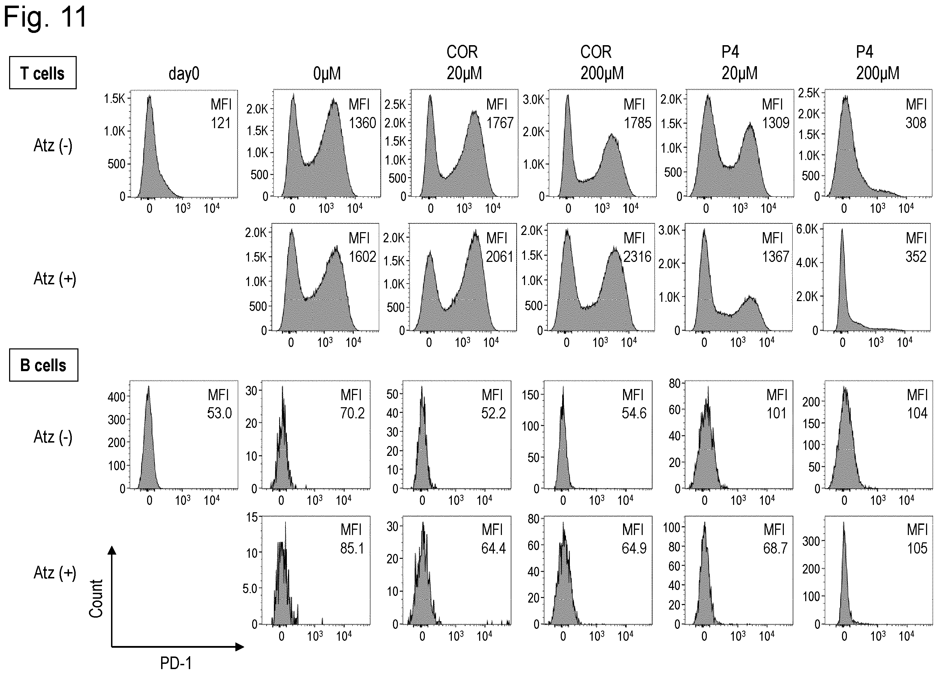

[Figure 11] Figure 11 illustrates the relationship between atezolizumab and PD-1 expression in a lymphocyte. Human PBMCs were cultured for 3 days in a medium supplemented with 0 to 200 µM P4/COR and 100 µg/mL atezolizumab, and expression of PD-1 on a lymphocyte was confirmed by FCM. Panels in upper two rows illustrate results obtained with a T cell (CD3 gated) and panels in lower two rows illustrate results obtained with a B cell (CD19 gated). Upper panels in each row illustrate results obtained without atezolizumab addition (Atz(-)), and lower panels illustrate results obtained with atezolizumab addition (Ats(+)). The ordinate indicates a count number, and the abscissa indicates expression level of PD-1.

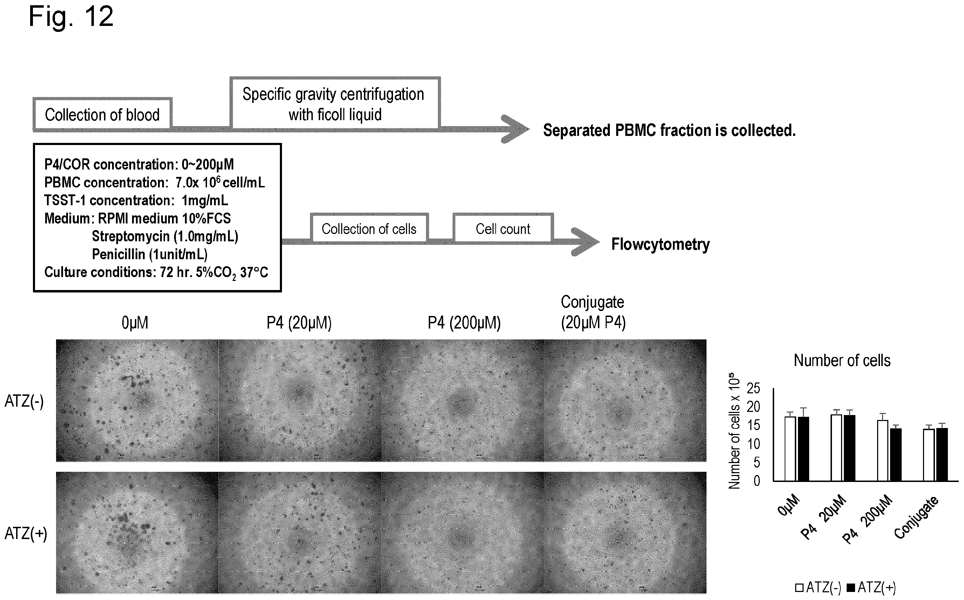

[Figure 12] Figure 12 illustrates PBMC activation regulation - 1: cell proliferation by atezolizumab and P4. Human PBMCs were cultured for 3 days in a medium supplemented with 0 to 200 µM P4 and 100 µg/mL of atezolizumab, or D2125 containing an equivalent of 20 µM P4, and change in the number of cells and the degree of aggregation through cell activation were confirmed. Upper left part illustrates a protocol, a lower left part provides photographs, and a right panel illustrates the number of cells on day 3. A significant difference test was conducted, but there was no significant difference.

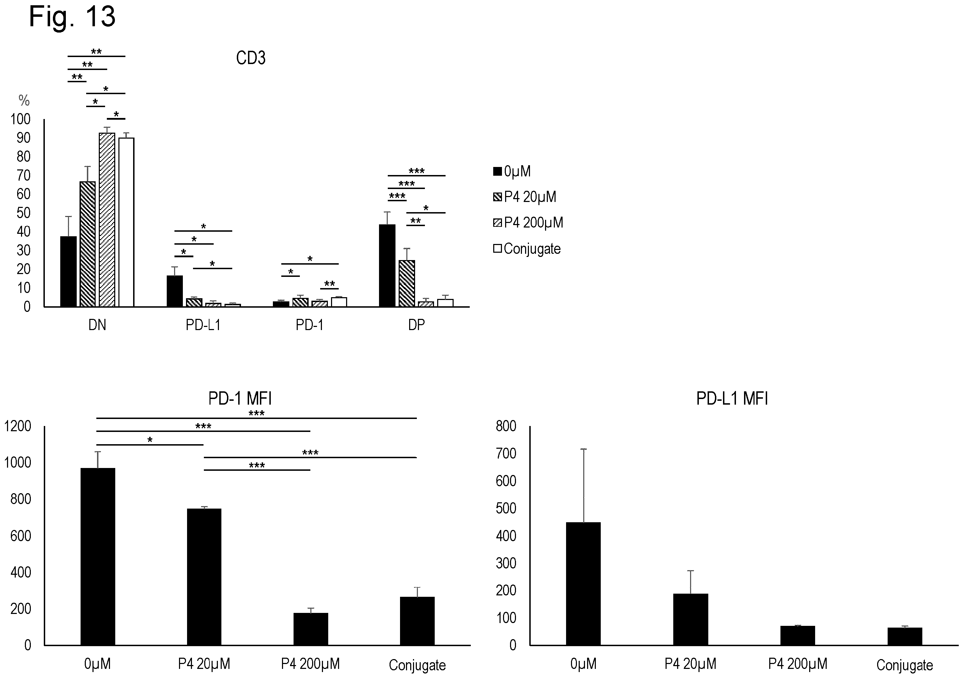

[Figure 13] Figure 13 illustrates PBMC activation regulation (T cell) by P4 and D2125. Human PBMCs were cultured for 3 days by the method illustrated in Figure 12, and expression of PD-1/PD-L1 on a lymphocyte was confirmed by FCM. An upper panel illustrates a ratio of PD-1 positive cells, PD-L1 positive cells, PD-1/PD-L1 negative cells, or PD-1/PD-L1 positive cells in CD3 gated cells, and panels of lower two rows illustrate expression levels (MFI) of PD-1 and PD-L1 in CD3 gated cells. According to a T test, p < 0.05*, p < 0.01**, and p < 0.005***.

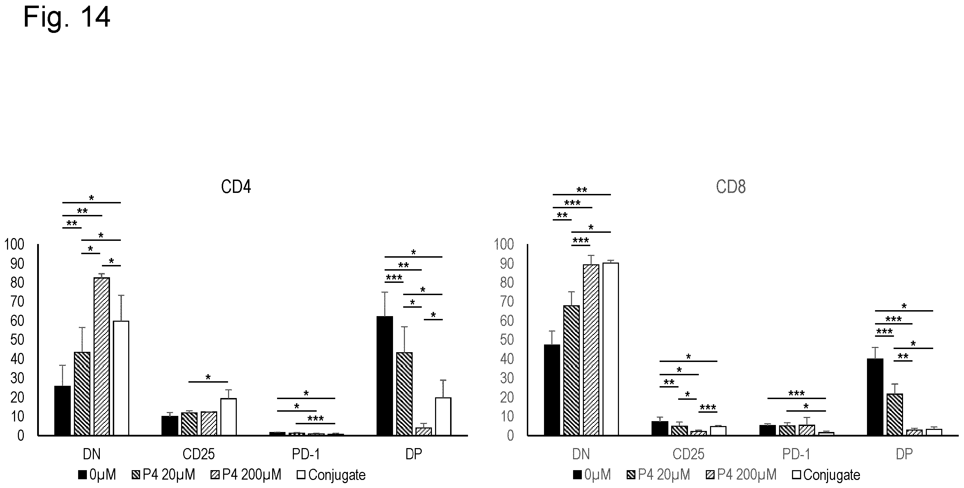

[Figure 14] Figure 14 illustrates PBMC activation regulation (CD4/CD8 T cell) by P4 and D2125. Human PBMC was cultured for 3 days by the method illustrated in Figure 11, and a ratio of PD-1 positive cells, PD-L1 positive cells, PD-1/PD-L1 negative cells, or PD-1/PD-L1 positive cells in CD3 and CD4 or CD8 gated cells is illustrated. Left panel: CD4. Right panel: CD8. According to a T test, p < 0.05*, p < 0.01**, and p < 0.005***.

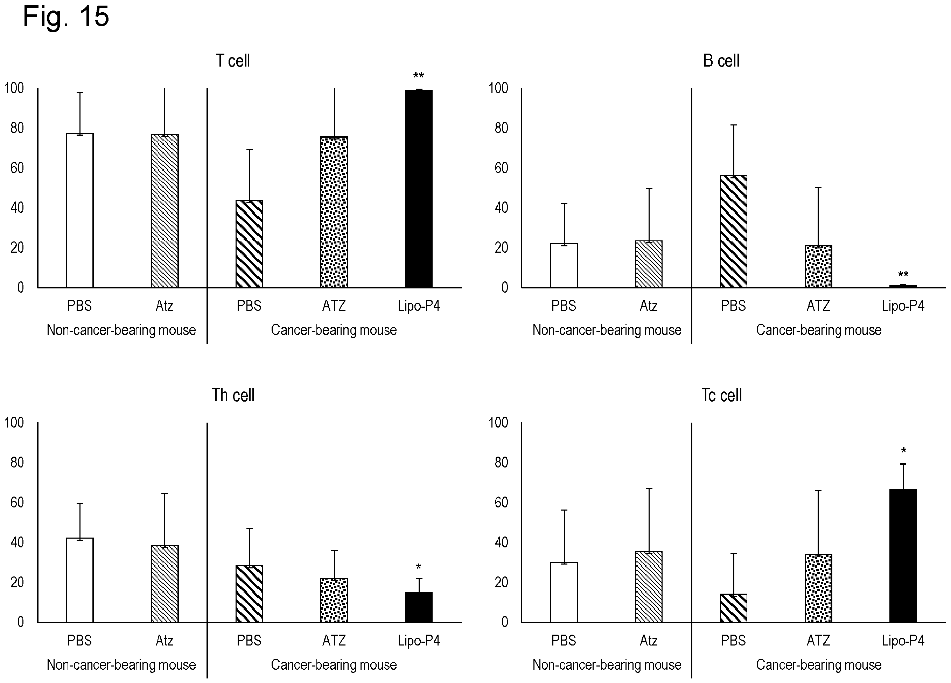

[Figure 15] Figure 15 illustrates results of measurement of a CD19 positive B cell, a CD3 positive T cell, a CD4 positive helper T cell, and a CD8 positive killer T cell obtained by administering atezolizumab (Atz), or liposome-encapsulated P4 bound to atezolizumab (Lipo-P4) to a non-cancer-bearing mouse (CMV-NOG-hIL-4-Tg mouse) or a cancer-bearing mouse (a CMV-NOG-hIL-4-Tg mouse to which human breast cancer cell line MDA-MB-231 and human PBMCs were transplanted). According to a T test, p < 0.05*, and p < 0.01**.

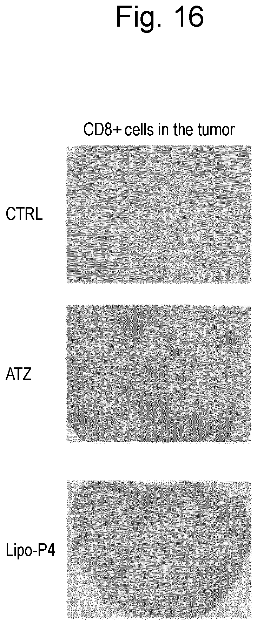

[Figure 16] Figure 16 illustrates results of observation of a tumor tissue by histochemical staining, obtained by administering atezolizumab (Atz) or liposome-encapsulated P4 bound to atezolizumab (Lipo-P4) to a cancer-bearing mouse (a CMV-NOG-hIL-4-Tg mouse to which human breast cancer cell line MDA-MB-231 and human PBMCs were transplanted).

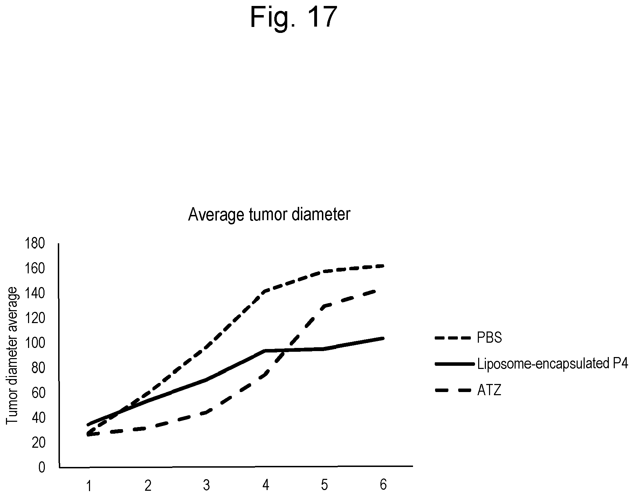

[Figure 17] Figure 17 illustrates results of measurement of a tumor diameter obtained by administering atezolizumab (ATZ) or liposome-encapsulated P4 bound to atezolizumab to a cancer-bearing mouse (a CMV-NOG-hIL-4-Tg mouse to which human breast cancer cell line MDA-MB-231 and human PBMCs were transplanted).

### Description of Embodiment

Hereinafter, the present invention will be described in more detail.

### <T Cell and/or B Cell Activity Regulator>

The T cell and/or B cell activity regulator of the present invention comprises progesterone or a derivative thereof as an active ingredient.

The T cell and/or B cell activity regulator of the present invention can reversibly act on a T cell and/or a B cell. The T cell and/or B cell activity regulator of the present invention can maintain expression of CD62L to localize a T cell in a lymph node. The T cell and/or B cell activity regulator of the present invention can reduce expression of PD-1 in a T cell and/or a B cell, and can also suppress expression of PD-L1 in a T cell and/or a B cell.

Progesterone or a derivative thereof may be either a natural compound or a synthetic compound, and may be any one of progesterone, a progesterone metabolite (such as 17α-hydroxyprogesterone), and an any other progestin. When progestin is used, a synthetic progesterone is preferably selected from the group consisting of a derivative of progesterone or testosterone, and a derivative of another molecule and/or compound having progestogen activity. A derivative refers to a chemical substance made of or providing a parent compound generated through one or a plurality of chemical reactions. The progestin may be any one of a natural progesterone, a synthetic progesterone, a natural or synthetic derivative of progesterone and/or another progestogen compound, and a combination thereof.

Specific examples of the progestin include, but are not limited to, 17α-hydroxyprogesterone caproate, medroxyprogesterone acetate, norethindrone, norethindrone acetate, norethindrone enanthate, desogestrel, levonorgestrel, lynestrenol, ethynodiol diacetate, norgestrel, norgestimate, norethynodrel, gestodene, drospirenone, trimegestone, levodesogestrel, gestodine, nestron, etonogestrel, and a derivative from 19-nortestosterone.

A method for administering the T cell and/or B cell activity regulator is not especially limited, and may be either oral administration or parenteral administration. Examples of the parenteral administration include, but are not limited to, intravenous administration, intraarterial administration, intraperitoneal administration, subcutaneous administration, and intramuscular administration. The T cell and/or B cell activity regulator of the present invention can be, for example, administered by injection. A solution for the injection can be formulated by using a carrier consisting of a salt solution, a glucose solution, a mixture of a brine and a glucose solution, or any of various buffers. Alternatively, it may be formulated in the form of a powder, so as to prepare an injection solution by mixing it with the liquid carrier at the time of use.

The T cell and/or B cell activity regulator of the present invention can be prepared by a known method using a pharmaceutically acceptable carrier in accordance with a dosage form. As the carrier, for example, an excipient, a binding agent, a disintegrating agent, a lubricant, a diluent, a dissolution assisting agent, a suspending agent, a tonicity agent, a pH adjustor, a buffer, a stabilizing agent, a colorant, a flavoring agent, an odor improving agent, or the like can be used.

In oral administration, an oral solution, a powder, a pill, a capsule, a tablet, or the like can be applied. An oral solution can be produced as an oral liquid preparation such as a suspending agent or a syrup by using water, a sugar such as sucrose, sorbitol, or fructose, a glycol such as polyethylene glycol, an oil such as sesame oil or soybean oil, a preservative such as alkyl parahydroxybenzoate, a flavor such as a strawberry flavor or peppermint, or the like. A powder, a pill, a capsule, and a tablet can be formulated by using an excipient such as lactose, glucose, sucrose, or mannitol, a disintegrating agent such as starch or sodium alginate, a lubricant such as magnesium stearate or talc, a binding agent such as polyvinyl alcohol, hydroxypropyl cellulose, or gelatin, a surfactant such as fatty acid ester, a plasticizer such as glycerin, or the like.

The T cell and/or B cell activity regulator of the present invention can be administered in a therapeutically effective amount. For example, it can be administered in a dose of 1 µg/kg of body weight to 1000 mg/kg of body weight per day.

The T cell and/or B cell activity regulator of the present invention can be used in combination treatment with an anti-human PD-L1 monoclonal antibody. Thus, the present invention provides a pharmaceutical composition comprising, as active ingredients, the T cell and/or B cell activity regulator of the present invention and an anti-human PD-L1 monoclonal antibody.

A monoclonal antibody means an antibody obtained from a substantially homogeneous antibody population. The monoclonal antibody may be a chimeric antibody. In a chimeric antibody, a part of a heavy chain and/or a light chain has a sequence being the same as or homologous to a corresponding sequence of an antibody derived from a specific species, or an antibody belonging to a specific antibody class or subclass, and the rest of the chain (one or a plurality) has a sequence being the same as or homologous to a corresponding sequence of an antibody derived from another species, an antibody belonging to another antibody class or subclass, or a fragment of such an antibody.

The monoclonal antibody may be prepared by a hybridoma method, or may be prepared by a recombinant DNA method. As the monoclonal antibody, a fragment thereof (such as F(ab')₂, Fab', Fab, Fv, or sFv) may be used.

The anti-human PD-L1 monoclonal antibody is preferably a humanized anti-human PD-L1 monoclonal antibody. In antibody humanization technology, a DNA sequence encoding one or a plurality of polypeptides of an antibody molecule is generally engineered by recombinant DNA technology. A humanized type non-human antibody (or a fragment thereof) is a chimeric antibody or chimeric antibody chain (or a fragment thereof such as sFv, Fv, Fab, Fab', or F(ab')₂, or another antigen-binding portion of the antibody) containing a part of an antigen-binding site derived from a non-human (donor) antibody incorporated into a framework of a human (recipient) antibody.

In order to produce a humanized antibody, a residue of one or a plurality of complementarity determining regions (CDRs) of a recipient (human) antibody molecule is replaced with a reside of one or a plurality of CDRs of a donor (non-human) antibody molecule known to have a desired antigen-binding property (such as a certain level of specificity to or affinity for a target antigen). In some cases, an Fv framework (FR) residue of a human antibody is replaced with a corresponding non-human residue. The humanized antibody can contain a residue found in neither the recipient antibody nor a CDR sequence and framework sequence to be introduced. The humanized antibody generally has one or a plurality of amino acid residues introduced from a supply source of a non-human. A humanized antibody usually refers to a human antibody in which a part of CDR residues, and possibly a part of FR residues is replaced with a residue at a similar site of a rodent antibody. A humanized antibody generally contains an antibody constant region (Fc), and usually at least a part of an Fc of a human antibody.

A method for humanizing a non-human antibody is known. For example, a humanized antibody can be produced by replacing corresponding sequences of a human antibody with (one or a plurality of) rodent CDRs or CDR sequences in accordance with a method of Winter et al., (Jones et al., Nature, 321: 522-525 (1986), Riechmann et al., Nature, 332: 323-327 (1988), or Verhoeyen et al., Science, 239: 1534-1536 (1988)).

Examples of the anti-human PD-L1 monoclonal antibody include human type PD-L1 monoclonal antibody, human atezolizumab (trade name: Tecentriq), human type PD-L1 monoclonal antibody, avelumab (trade name: Bavencio), and human type PD-L1 monoclonal antibody, durvalumab (trade name: Imfinzi), and atezolizumab is particularly preferable.

The amount of the anti-human PD-L1 monoclonal antibody to be used varies depending on the type of the anti-human PD-L1 monoclonal antibody used in combination, the administering method, the symptoms, the age and the like of a patient, and for example, can be 0.01 mg/kg of body weight (preferably 0.1 mg/kg of body weight) or more and 1000 mg/kg of body weight (preferably 100 mg/kg of body weight) or less per dose for oral administration, and can be 0.001 mg/kg of body weight (preferably 0.01 mg/kg of body weight) or more and 1000 mg/kg of body weight (preferably 100 mg/kg of body weight) or less per dose for intravenous administration. The frequency of the administration can be once to several times a day.

An example of the pharmaceutical composition of the present invention provides an immunoliposome comprising a liposome comprising the T cell and/or B cell activity regulator of the present invention, wherein the anti-human PD-L1 monoclonal antibody or the anti-human PD-1 monoclonal antibody is bound to a membrane of the liposome.

A liposome refers to a lipid structure formed from an amphipathic vesicle-forming lipid. A liposome is typically a closed vesicle containing a single layer or multilayer of a lipid bilayer having an aqueous phase inside. A lipid bilayer refers to a structure in which hydrophobic regions of polar lipid molecules are associated with each other to align a hydrophobic portion toward the center of the bimolecular layer and a hydrophilic region toward the aqueous phase. An immunoliposome refers to a complex formed from a liposome and a protein (such as an antibody).

The liposome used in the present invention preferably comprises an amphipathic vesicle-forming lipid. A lipid component comprised in a liposome includes a phospholipid, a glycolipid, a sphingolipid, a sterol, a glycol, a saturated or unsaturated fatty acid, a surfactant, a derivative lipid having a hydrophilic polymer, and the like.

The phospholipid is roughly divided into a glycerophospholipid and a sphingophospholipid. A representative example of the glycerophospholipid includes a phospholipid having at least one head group, such as phosphatidylcholine (PC), phosphatidylserine (PS), phosphatidylinositol (PI), phosphatidylglycerol (PG), phosphatidylethanolamine (PE), or phosphatidic acid (PA). On the other hand, a representative example of the sphingophospholipid includes sphingomyelin.

Among those described above, examples of phosphatidylcholines include dipalmitoylphosphatidylcholine (DPPC), distearoylphosphatidylcholine (DSPC), dimyristoylphosphatidylcholine (DMPC), dioleoylphosphatidylcholine (DOPC), dilauroylphosphatidylcholine (DLPC), didecanoylphosphatidylcholine (DDPC), dioctanoylphosphatidylcholine (DOPC), dihexanoylphosphatidylcholine (DHPC), dibutyrylphosphatidylcholine (DBPC), dielaidoylphosphatidylcholine, dilinoleoylphosphatidylcholine, diarachidonoylphosphatidylcholine, diicosenoylphosphatidylcholine (DEPC), diheptanoylphosphatidylcholine, dicaproylphosphatidylcholine, diheptadecanoylphosphatidylcholine, dibehenoylphosphatidylcholine, eleostearoylphosphatidylcholine, hydrogenated egg phosphatidylcholine (HEPC), hydrogenated soy phosphatidylcholine (HSPC), 1-palmitoyl-2-arachidonoyl phosphatidylcholine, 1-palmitoyl-2-oleoyl phosphatidylcholine, 1-palmitoyl-2-linoleoyl phosphatidylcholine, 1-palmitoyl-2-myristoyl phosphatidylcholine, 1-palmitoyl-2-stearoyl phosphatidylcholine, 1-stearoyl-2-palmitoyl phosphatidylcholine, 1,2-dimyristoylamide-1,2-deoxyphosphatidylcholine, 1-myristoyl-2-palmitoyl phosphatidylcholine, 1-myristoyl-2-stearoyl phosphatidylcholine, di-O-hexadecylphosphatidylcholine, transdielaidoylphosphatidylcholine, dipalmiterydoyl-phosphatidylcholine, n-octadecyl-2-methylphosphatidylcholine, n-octadecylphosphatidylcholine, 1-laurylpropanediol-3-phosphocholine, erythro-N-lignoceroyl sphingophosphatidylcholine, and palmitoyl-(9-cis-octadecenoyl)-3-sn-phosphatidylcholine.

Examples of phosphatidylethanolamines (cephalins) include dipalmitoyl phosphatidylethanolamine (DPPE), distearoyl phosphatidylethanolamine (DSPE), dioleoyl phosphatidylethanolamine (DOPE), dilauroyl phosphatidylethanolamine (DLPE), dimyristoyl phosphatidylethanolamine (DMPE), didecanoyl phosphatidylethanolamine (DDPE), N-glutaryl phosphatidylethanolamine (NGPE), lisophosphatidylethanolamine, N-(7-nitro-2,1,3-benzoxydiazol-4-yl)-1,2-dioleoyl-sn-phosphatidylethanolamine, eleostearoyl phosphatidylethanolamine, N-succinyldioleoyl phosphatidylethanolamine, and 1-hexadecyl-2-palmitoylglycerophosphatidylethanolamine.

As the glycolipid, a glyceroglycolipid, a sphingoglycolipid, or another glycolipid can be used. As the sterol, cholesterol or the like can be used. As a neutral lipid, a diglyceride (such as diolein or dipalmitrain) or the like can be used.

As the saturated or unsaturated fatty acid, caprylic acid, pelargonic acid, capric acid, undecylenic acid, lauric acid, tridecylenic acid, myristic acid, pentadecylenic acid, palmitic acid, margaric acid, stearic acid, nonadecylenic acid, arachidic acid, dodecenoic acid, tetradecenoic acid, oleic acid, linolic acid, linoleic acid, eicosenoic acid, erucic acid, docosapentaenoic acid, or the like can be used. As a charged lipid, an anionic lipid, a cationic lipid or the like can be used. As the surfactant, a cationic surfactant, an anionic surfactant, an amphoteric surfactant, or a nonionic surfactant can be used.

An example of a derivative lipid having a hydrophilic polymer includes one containing any of the above-described lipids and a hydrophilic polymer. The lipid and the hydrophilic polymer are bound to each other through a covalent bond formed directly or via a linker by a functional group of the lipid and a functional group of the hydrophilic polymer.

Examples of a lipid derivative of a hydrophilic polymer include, but are not limited to, a polyethylene glycol modified lipid, a polyethylene imine derivative, a polyvinyl alcohol derivative, a polyacrylic acid derivative, a polyacrylamide derivative, a dextran derivative, a polyglycerin derivative, a chitosan derivative, a polyvinyl pyrrolidone derivative, a polyaspartic acid amide derivative, a poly-L-lysine derivative, a mannan derivative, and a pullulan derivative.

In the present invention, an anti-human PD-L1 monoclonal antibody or an anti-human PD-1 monoclonal antibody can be bound to the membrane of the liposome. An anti-human PD-L1 monoclonal antibody or an anti-human PD-1 monoclonal antibody can be bound to the membrane of the liposome by adding the anti-human PD-L1 monoclonal antibody or the anti-human PD-1 monoclonal antibody to a liposome solution to be reacted.

As a constituent of the immunoliposome, a functionality imparting agent may be further added. Examples of the functionality imparting agent include a membrane stabilizing agent, an agent for adjusting hydrophilicity on a membrane surface, a curvature adjuster, an antioxidant, a charge imparting agent, and a cryoprotective agent. For example, a sugar, a glycolipid, glycerin, a stabilizer such as polyethylene glycol, and an antioxidant such as tocopherol or ascorbic acid can be used. Cholesterols can be used as a membrane stabilizer, an agent for adjusting hydrophilicity on a membrane surface, or a curvature adjuster for liposome, and tocopherols can be used as an antioxidant. Any of other compounds stabilizing a liposome can be used instead of a cholesterol. Other liposome stabilizing compounds are known in this field. For example, a saturated phospholipid produces a liposome having a high transition temperature. In order to avoid limitation of electrostatic association between an antigen and a liposome, the antibody and progesterone may be sequestered in the liposome.

The present invention also provides a pharmaceutical composition comprising a therapeutically effective amount of immunoliposome, and pharmaceutically acceptable diluent, carrier, solubilizing agent, emulsifier, preservative and/or auxiliary agent. The pharmaceutical composition of the present invention can contain a substance for formulation for changing, maintaining, or holding pH, an osmotic pressure, viscosity, transparency, color, isotonicity, sterility, stability, a solubility rate, a sustained release rate, an absorption rate, or a penetration rate.

Examples of the substance for formulation include, but are not limited to, a natural lipid, a synthetic lipid, a sphingolipid, an ether lipid, a sterol, a cardiolipin, a cationic lipid, and a lipid modified with poly(ethylene glycol) or another polymer. Examples of the synthetic lipid include the following fatty acid components: lauroyl, myristoyl, palmitoyl, stearoyl, arakidoyl, oleoyl, linoleoyl, erucoyl, and a combination of these fatty acids. Other examples include the following: an amino acid such as glycine, alanine, glutamine, asparagine, arginine, or lysine; an antioxidant such as an antibacterial agent, ascorbic acid, sodium sulfate, or sodium hydrogen sulfite; a buffer such as phosphoric acid, citric acid, a borate buffer, hydrogen carbonate, or a Tris-hydrochloric acid (Tris-Hcl) solution; a filler such as mannitol or glycine; a chelating agent such as ethylenediamine tetraacetic acid (EDTA); a complexing agent such as caffeine, polyvinylpyrrolidine, β-cyclodextrin, or hydroxypropyl-β-cyclodextrin; an extending agent such as glucose, mannose, or dextrin; a monosaccharide, a disaccharide, glucose, mannose, and another hydrocarbon such as dextrin; a colorant, a flavoring agent, a diluent, an emulsifier, a hydrophilic polymer such as polyvinylpyrrolidine, a low molecular weight polypeptide, a salt-forming counter ion, benzalkonium chloride, benzoic acid, salicylic acid, thimerosal, phenetyl alcohol, a preservative such as methylparaben, propylparaben, chlorhexidine, sorbic acid, or hydrogen peroxide, a solvent such as glycerin, propylene glycol, or polyethylene glycol, a sugar alcohol such as mannitol, or sorbitol, a suspending agent, PEG, sorbitan ester, a polysorbitate such as polysorbitate 20 or polysorbitate 80, triton, toromethamine, a surfactant such as lecithin or cholesterol, a stabilization enhancer such as sucrose, or sorbitol, an elasticity enhancer such as sodium chloride, potassium chloride, or mannitol/sorbitol, a transport agent, a diluent, an excipient, and/or a pharmaceutical auxiliary agent.

The amount of such a substance for formulation to be added is preferably a 0.01 to 100-fold amount, and particularly preferably a 0.1 to 10-fold amount relative to the weight of the immunoliposome. An excipient and a carrier contained in the pharmaceutical composition may be a liquid or a solid. The excipient and the carrier may be water for injection or saline. The pharmaceutical composition can be prepared as an appropriate drug, a freeze-dry product, or a liquid having a selected composition and a necessary purity.

The T cell and/or B cell activity regulator, the agent for combination use, and the pharmaceutical composition of the present invention can be used for one or more selected from carcinoma, sarcoma, lymphoma, leukemia, myeloma, germinoma, brain tumor, carcinoid, neuroblastoma, retinoblastoma, and nephroblastoma. Specifically, examples of carcinoma include kidney cancer, malignant melanoma (melanoma), spinous cell carcinoma, basal cell carcinoma, conjunctival cancer, oral cancer, laryngeal cancer, pharyngeal cancer, thyroid cancer, lung cancer, breast cancer, esophageal cancer, stomach cancer, duodenal cancer, small intestine cancer, large intestine cancer, rectal cancer, appendix cancer, anal cancer, fiver cancer, gallbladder cancer, bile duct cancer, pancreatic cancer, adrenal cancer, bladder cancer, prostate cancer, uterine cancer, and vaginal cancer. Examples of sarcoma include liposarcoma, angiosarcoma, chondrosarcoma, rhabdomyosarcoma, Ewing sarcoma, osteosarcoma, undifferentiated pleomorphic sarcoma, myxofibrosarcoma, malignant peripheral nerve sheath tumor, retroperitoneal sarcoma, synovial sarcoma, uterine sarcoma, gastrointestinal stromal tumor, leiomyosarcoma, and epitheliod sarcoma. Examples of lymphoma include B cell lymphoma, T/NK cell lymphoma, and Hodgkin lymphoma. Examples of leukemia include myelocytic leukemia, lymphatic leukemia, a myeloproliferative disease, and myelodysplastic syndromes. An example of myeloma includes multiple myeloma, examples of germinoma include testicular cancer, and ovarian cancer, and examples of brain tumor include glioma, and meningioma. These are used particularly preferably for breast cancer, lung cancer (particularly non-small cell lung cancer, or small cell lung cancer), hepatocellular cancer, urothelial cancer, and malignant melanoma (melanoma).

The present invention will now be more specifically described with reference to Examples below, but it is noted that the present invention is not limited to these Examples.

### Examples

### [Example 1]

### Cell Line and Mouse

All of JEG-3, HEK293, BeWo, P3X and A20 used here were ones stored in Department of Molecular Life Sciences, Basic Medical Science and Molecular Medicine, Tokai University School of Medicine. JEG-3, HEK293, and BeWo were cultured using D-MEM, and P3X and A20 were cultured using RPMI 1640. NOG mice were purchased from Invivo Science (Kawasaki, Japan). NOG-hIL-4-Tg mice used here were ones maintained in an isolator of Institute for Experimental Animals, Tokai University School of Medicine. From these mice, individuals having a human IL-4 concentration of 100 pg/mL or more were selected by DNA typing and ELISA to be used for transplantation.

Human peripheral blood mononuclear cells (PBMCs) were prepared as follows.

30 mL of heparinized peripheral blood was collected from a healthy donor with Vacutainer ACD Tubes (NIPRO Corporation, Japan, Osaka). Immediately after that, the collected peripheral blood was layered on Ficoll-Hypaque (SIGMA-ALDRICH, UK, London), and a mononuclear cell fraction was collected by specific gravity centrifugation (500 × g, 30 minutes, 20°C). The resultant cells were washed with PBS by centrifugation at 300 × g for 5 minutes at 4°C, and then the number of the cells was counted for use.

The various cell lines were cultured as follows.

Each of human choriocarcinoma cell line JEG-3, BeWo, and embryonic kidney cell line HEK293 was cultured for 10 days at 37°C in 5% CO₂ in a 10% Fetal Calf Serum (FCS)-containing DMEM (Gibuco) to which water-soluble progesterone (P4; water soluble) (Sigma) or cortisol (COR) (Sigma) was added in various concentrations. Each of mouse myeloma cell line P3-X63-Ag8-U1 (P3X) and mouse lymphoma cell line A20 was cultured for 3 days at 37°C in 5% CO₂ in 10% FCS-containing RPMI 1640 to which P4 or COR was added in various concentrations. Proliferation of the cells was evaluated by measuring the number of the cells.

Human peripheral blood mononuclear cells (PBMCs) were cultured as follows.

The PBMCs (final concentration: 1 × 10⁶/mL) were cultured for 3 days at 37°C in 5% CO₂ in 10% FCS-containing RPMI 1640 to which P4 or COR was added in various concentrations with stimulation by 1 µg/mL of toxic shock syndrome toxin-1 (TSST-1). These cells were collected, washed with PBS, and dispensed into a Fisher tube in an amount of 1 × 10⁶ cells/tube. For analysis of a human cell surface marker, a fluorescent-labeled anti-human monoclonal antibody (mAbs) was used. The cells were stained with the mAbs, a reaction was performed for 15 minutes at 4°C, and the resultant was washed with PBS containing 1% (w/v) BSA. Then, the resultant was analyzed by FACS Fortessa, or FACS Verse (BD Bioscience, Franklin Lakes, NJ). In the analysis, a leukocyte gated or lymphocyte gated cells were further gated by expression of human CD45, and then the analysis was performed. A list of antibodies used here is shown in Table 1. A culture supernatant was collected, and 25 µL thereof was dispensed to quantitatively determine cytokine with Legend plexTM kit from BioLegend.

**[Table 1]**

| Antibody | Clone | Company |
|---|---|---|
| FITC anti-human CD3 | UCHT1 | BioLegend |
| APC anti-human CD4 | RPA-T4 | BioLegend |
| BrilliantViolet510^{™} anti-human CD4 | OKT4 | BioLegend |
| PE/Cy7 anti-human CDS | UCHT2 | BioLegend |
| Alexa Fluor^{®}700 anti-human CD8 | HIT8a | BioLegend |
| Pacific Blue^{™} anti-human CD8a | RPA-T8 | BioLegend |
| APC/Cy7 anti-human CD19 | HIB19 | BioLegend |
| PE anti-human CD25 | BC96 | BioLegend |
| PE/Cy7 anti-human CD25 | BC96 | BioLegend |
| PE anti-human CD27 | M-T271 | BD Bioscience |
| Alexa Fluor^{®}700 anti-human CD38 | HIT2 | BioLegend |
| Pacific Blue^{™} anti-human CD45 | HI30 | BioLegend |
| BrillianViolet650^{™} anti-human CD45RA | HI100 | BioLegend |
| APC anti-human CD45RA | HI100 | BioLegend |
| APC/Cy7 anti-human CD45RO | UCHL1 | BioLegend |
| PE/Cy7 anti-human CD56 | NCAM16.2 | BD Bioscience |
| PE anti-human CD62L | DREG-56 | BioLegend |
| BUV395 anti-human CD274 (PD-L1) | MIH1 | BD Bioscience |
| PerCP/Cy5.5 anti-human CD279 (PD-1) | EH12.2H7 | BioLegend |
| FITC anti-human IgD | IA6-2 | BD Bioscience |

| Company | Address |
|---|---|
| BD Bioscience | 1 Becton Drive, Franklin Lakes, NJ |
| BioLegend | 9727 Pacific Heights Blvd, San Diego, CA |
| eBioscience | 10255 Science Center Drive San Diego, CA |

In a restimulation experiment, PBMCs were cultured for 6 hours at 37°C in 5% CO₂ in 10% FCS-containing RPMI 1640 to which 200 µM P4 or COR was added. These cells were washed, adjusted to a final concentration of 1 × 10⁶/mL, and cultured again in the presence or absence of 200 µM P4 or COR with stimulation by 1 µM TSST-1. After 72 hours, the resultant cells were collected to be analyzed by FCM.

As for an experiment of atezolizumab addition, 0 to 200 µM P4 or COR, 1 µM TSST-1, and 100 µg/mL atezolizumab were added to PBMCs, and the resultant was cultured for 72 hours at 37°C in 5% CO₂, followed by analysis by FCM.

Transplantation to a PBMC-NOG-hIL-4-Tg mouse, and analysis of an engrafted human cells were performed as follows.

PBMCs were cultured for 6 hours at 37°C in 5% CO₂ in 10% FCS-containing RPMI 1640 to which 200 µM P4 or COR was added. These cells were collected, and washed, and 2.5 × 10⁶ PBMCs were intravenously transplanted to each 8- to 9-week-old NOG-hIL-4-Tg mouse (a hIL-4 concentration in blood of 100 pg/mL or more).

A CH401MAP peptide is a 20-mer MAP peptide containing a partial sequence of HER2/neu as an epitope of an anti-HER2 monoclonal antibody (Miyako H., et al., Anticancer Res (2011) 31(10): 361-368). This peptide was produced by using T Rink amide resin (0.4 to 0.7 mmol/g) with ACT357 peptide synthesizer (Advanced Chemtech, Louisville, KY). This was mixed with Freund's complete adjuvant (CFA) (Wako Pure Chemical Industries, Ltd., Osaka, Japan) (50 g/head, 100 µl 1:1/v:v) in equivalent amounts to obtain an emulsion, which was administered to the abdominal cavity of a PBMC-NOG-hIL-4-Tg mouse. As a negative control, an equivalent amount of PBS was formed into an emulsion, and similarly administered to a mouse. Boost immunization was performed using Freund's incomplete adjuvant (IFA) (Wako Pure Chemical Industries, Ltd.) 2 weeks after the initial immunization. After 2 weeks, the mouse was anesthetized to collect heparinized blood, and was sacrificed. Human leukocyte fractions of various lymph tissues collected were subjected to FCM analysis. Besides, a plasma component was used for measuring an antibody titer by ELISA, and a spleen cell was used for producing a hybridoma.

A hybridoma was prepared from a spleen cell of the PBMC-NOG-hIL-4-Tg mouse as follows. Spleen cells were prepared from an immunized humanized NOG-hIL-4-Tg mouse, hemolyzed, and then washed with PBS. The cells were mixed with P3X to be fused by an electrical cell fusion method using BEX CFB16-HB (BEX Co., Ltd., Tokyo, JPN) and an electrode, LF497P2. Conditions for the electrical cell fusion method were AC 30 [V], 20 [s], DC 350 [V], 30 µs/500 ms, DC cycle 3, AC 30 [V], 7 [s], Fade on, in electro fusion buffer (0.3 M mannitol, 0.1 mM calcium chloride and/or 0.1 mM magnesium chloride). The fused cells were cultured in HAT medium for 2 weeks. The amount of the antibody in a culture supernatant was quantitatively determined by ELISA.

The quantitative determination of a protein by ELISA was performed as follows.

The amount of human IL-4 protein was determined with Human IL-4 ELISA Set BD OptEIATM (BD Biosciences). The quantitative determination of an IgG antibody was performed in accordance with previously reported paper (Kametani Y., et al., Exp Hematol (2006) 34(9): 1240-1248). Wells of a microtiter plate (Sumiron, Tokyo, Japan) were coated with CH401MAP peptide dissolved in carbonate buffer (pH 9.5) to allow the antigen to be adsorbed to the plate overnight at 4°C. The wells were then washed with PBS-Tween (0.05% v/v), and reacted with 3% BSA-PBS at room temperature (RT) for 2 hours. After washing the resultant with PBS-Tween 3 times, a mouse plasma was added thereto in 10-fold dilution series, followed by a reaction at RT for 2 hours. The resultant plate was washed 3 times, and biotin-conjugated mouse anti-human IgG mAb (BD Pharmingen, San Diego, USA) (1:3,000) was added thereto. The resultant plate was subjected to a reaction at 37°C for 2 hours, and then washed 3 times, and streptavidin-horseradish peroxidase (1:50,000 v/v; BD Pharmingen) was added thereto. The resultant plate was subjected to a reaction at RT for 1 hour, and then washed, and EIA substrate kit solution (Bio-Rad Laboratories, Hercules, CA, USA) was added thereto. The reaction was stopped with 10% HCl, and an absorbance at 450 nm was measured.

Analysis of graft versus host disease (GVHD) model of NOG was performed as follows.

In the analysis using the GVHD model, PBMCs prepared in the same manner as described above were washed with PBS, and 2.5 × 10⁶ PBMCs were intravenously transplanted to each 8- to 9-week-old NOG mouse. P4 (2 mg/head) or COR (2 mg/head), or, as a negative control, PBS was subcutaneously administered twice a week (every 3 or 4 days). At the same time as the administration, the weight was measured. After 4 weeks, the mouse was anesthetized to collect heart blood, and euthanized. From various lymphoid organs excised, human cells were prepared, hemolyzed, and then subjected to FCM. Besides, the lung and the liver were excised, and immunohistochemically stained as follows.

A tissue of the mouse was immobilized with 20% formalin (Wako Pure Chemical Industries, Ltd.), and embedded in paraffin. The obtained paraffin block was sliced, and the resultant was subjected to deparaffinization, mounted on a slide glass, and stained with hematoxylin and eosin (HE). In immunohistochemical staining with an anti-human CD45 antibody, the slide was subjected to a heat treatment at 97°C for 20 minutes, and subjected to an endogenous peroxidase treatment through a reaction with 0.3% H₂O₂/MetOH at room temperature for 10 minutes. Thereafter, the resultant was blocked with 1% goat serum, and anti-human CD45 (Dako) was added thereto, followed by a reaction overnight at 4°C. The resultant slide was washed 3 times with 0.01 M PBS for 5 minutes, and colored with a DAB solution. The resultant was washed with running water for 3 minutes, and stained with hematoxylin, followed by dehydration and penetration/encapsulation.

A conjugate of P4-encapsulated liposome and atezolizumab was produced as follows. To 2.0 mg/mL atezolizumab (in 10 mM PBS buf, pH 7.4), 500 mM EDTA and 2-mercaptoethanol were added respectively at final concentrations of 5 mM and 50 mM, followed by stirring. After performing a reaction at 37°C for 90 minutes, the resultant was subjected to ultrafiltration (Amicon Ultra 15 10 KDa) with 10 mM HEPES buf, pH 7.2 (containing 5 mM EDTA) to remove 2-mercaptoethanol. The resultant was treated with a 0.22 µm filter (diameter: 13 mm).

A progesterone-encapsulated liposome was prepared as follows.

A rotor was put in a 50 mL eggplant flask, and a lipid (DSPC: 42.5 mg DSPE-PEG2000 MA: 13.8 mg) was dissolved in 7 mL of tBuOH melted at 50°C, followed by stirring for 5 minutes in a water bath at 50°C. To the resultant, 3.5 mL of a 4.0 mg/mL progesterone solution (dissolved in tBuOH) was added, and the resultant was stirred for 5 minutes in a water bath at 50°C, and frozen for 5 minutes with liquid nitrogen to be freeze-dried. To the resultant, 7 mL of 10 mM HEPES and 150 mM NaCl (pH 7.2) were added, followed by stirring at 37°C for 1 hour. The resultant was subjected to an ultrasonic treatment with a bath-type ultrasonic device, and sampled before starting the treatment and every 30 minutes to measure a particle size/zeta potential. The treatment was completed when the measurements of the particle size reached one peak, or in 2 hours (30 minutes × 4 times) if one peak was not reached, and the next step was performed. An extruder treatment was performed (once each at 400 nm, 200 nm, and 100 nm).

The liposome was modified with an antibody as follows.

To 2.5 mL of the liposome solution, 750 µg of reduced atezolizumab was added, and the resultant was reacted under stirring at room temperature for 2 hours, followed by stirring overnight at 4°C. A 10 mL ultrafiltration cell was used for performing ultrafiltration (CAT: PBMK02510, pore size: 300 Kda). At that time, the ultrafiltration was repeated until the amount of a waste liquid became a 4-fold or more relative to the amount of the sample used in the ultrafiltration. The resultant was subjected to a filtration treatment with a 0.22 µm filter (diameter: 13 mm). For the obtained sample, quantitative determination of a lipid, quantitative determination of a protein, measurements of the particle size, the zeta potential, and the progesterone concentration were performed, and the following results were obtained.

**[Table 2]**

| Lipid Concentration (mg/mL) | Average Particle Size (nm) | Zeta Potential (mV) | Progesterone Concentration (µg/mL) | Antibody Concentration (mg/mL) |
|---|---|---|---|---|
| 26.06 | 112. 5 | -34.8 | 248.5 | 1.45 |

Statistical processing was performed with Microsoft Excel (Microsoft, Redmond, WA). Data was indicated as mean ± SD. A significant difference test was performed by two-sided Student's t-test analysis.

### [Results]

P4 and COR have been reported to bind to PgR in steroid hormone receptor-positive Luminal type cancer to support progression of the tumor, but on the other hand, have been occasionally reported to exhibit an anticancer effect against epithelial carcinomas including a breast cancer cell line (Yahya S., et al., Steroids (2017) 126: 15-23, You S., et al., Int J Nanomedicine (2010) 5: 221-229). Therefore, it was first examined what effect P4 exhibits against a cancer cell-derived cell line. JEG-3, BeWo, P3X, A20, and HEK293 were cultured in the presence of progesterone (P4) or cortisol (COR) used for suppressing ICI side effects to confirm concentration dependent change in the proliferating ability (Figure 1).

As a result, regarding JEG-3, BeWo, and HEK293, the cell proliferating ability thereof was minimally changed even by addition of 200 µM or less COR, but all of these cell lines little proliferated in the presence of 200 µM P4. On the contrary, regarding lymphoid lines P3X and A20, the proliferation was almost completely suppressed by addition of 2 µM or less COR, but the proliferation suppression was not observed by addition of 20 µM or less P4, and the proliferation suppression was observed by addition of P4 only at a high concentration of 200 µm. These results show that COR is more effective than P4 for suppressing lymphoid lines, but P4 can be more effective for suppressing epithelial cancers such as choriocarcinoma.

Therefore, peripheral blood mononuclear cells (PBMCs) of a healthy person were cultured in the presence of a superantigen TSST-1, and P4 or COR, to confirm change in the proliferating ability as well as to reveal cell activation/exhaustion level based on analysis of cell membrane surface antigens by flowcytometry (FCM).

As a result of measurement of the number of cells, it was revealed that the proliferation is suppressed by high concentration P4, but substantially no difference in the number of cells was caused by COR from that of a control group (Figure 1). Therefore, the activation/exhaustion level was studied. As a result, substantially no CD25-positive PD-1-positive later-phase activated T cells were observed in fresh PBMCs, but in a control group on day 3 of the culture, the ratio of such T cells was increased in both CD4 T cells and CD8 T cells (Figure 2).

In the presence of 20 to 200 µM P4, however, the ratio of the later-phase activated T cells was reduced dependent on the P4 concentration in both the CD4 and CD8 T cells, and the later-phase activated T cells were minimally detected at a concentration of 200 µM. Besides, activation marker-negative T cells were increased depending on the P4 concentration. On the other hand, in the presence of 20 to 200 µM COR, the change in the ratio of the later-phase activated T cells did not depend on the COR concentration in both the CD4 and CD8 T cells, and it was revealed that PD-1 expression was not suppressed depending on the COR concentration. On the other hand, the number of non-activated T cells was not largely increased, and thus it was revealed that the number of activated cells was reduced (data not shown). Based on these results, it was suggested that P4 suppresses activation/exhaustion of a T lymphocyte more strongly than COR, depending on the concentration. On the other hand, a B cell minimally expressed PD-1, and the expression was not increased even by TSST-1 stimulation and P4/COR stimulation (data not shown).

It is already well known that COR suppresses production of inflammatory cytokines, but it is not known to what extent P4 suppresses production of various cytokines. Therefore, some of the above-described culture supernatants were used for analyzing influence of P4 on cytokine production. As a result, for all the 10 cytokines illustrated in Figure 4, suppression of cytokine production was observed depending on the P4 concentration.

Therefore, it was studied how an activation marker changed between a case where P4 or COR was continuously added and a case where it was transiently added. We confirmed how much CD25+ PD-1+ cells expressed PD-L1 that is a ligand of PD-1. As a result, it was confirmed that most of the CD25+ PD-1+ cells were simultaneously PD-L1 positive (Figure 3). Therefore, it was decided to analyze increase of exhaustion signals between lymphocytes by comparing a ratio of PD-1/PD-L1-positive cells in P4-treated and COR-treated cells. As a result, when P4 was continuously added, the ratio of later-phase activated cells was remarkably reduced in both CD4 T cells and CD8 T cells, but when it was transiently added, exhibited activation was substantially equivalent to or greater than that of a control group. On the contrary, when COR was transiently added, CD8 T cell activation was significantly suppressed as compared with that in a control group, but on the other hand, even when COR addition was continued, the ratio of later-phase activated cells was not suppressed as much as that obtained by P4 addition (Figure 5).

On the other hand, in a B cell, the addition of COR tended to reduce PD-L1 expression, and continuous administration of P4 suppressed PD-L1 expression; however, the effect of P4 was lower than that in a T cell, and it was observed that transient addition thereof tended to maintain the expression (Figure 6). Besides, transient addition of P4 tended to rather increase PD-L1 expression.

Therefore, a GVHD onset model system obtained by transplanting human PBMCs to a NOG mouse was used to examine what *in vivo* effect was exhibited by P4 and COR as immune response to the xenograft (Figure 7). Human PBMCs were transplanted to a NOG mouse to obtain Hu-PBMC-NOG, and P4 was continuously injected to Hu-PBMC-NOG right from the transplantation. As a result, the number of spleen cells rather increased as compared with that in a control (left pane in Figure 7), and CD8 T cells were also observed to tend to increase, but weight loss by GVHD was only slightly caused at an initial stage, and substantially no symptoms appeared. Lymphocyte infiltration into a peripheral tissue such as the lung was different from that of a non-transplanted mouse but tended to be suppressed, and symptoms were remarkably relieved (right panel in Figure 7). On the other hand, it was revealed that administration of COR reduced lymphocytes and reduced GVHD symptoms, but lymphocyte engraftment failure was also caused.

Next, in order to confirm the effect of P4/COR on the antibody production ability, PBMCs of a healthy person were cultured *in vitro* in the presence of 200 µM P4 or COR for 6 hours in 5% CO₂ at 27°C, and the resultant was intravenously transplanted into a NOG-hIL-4-Tg mouse, and the mouse was immunized with 50 µg of CH401MAP emulsified with a Freund's adjuvant. After 2 weeks, boost immunization was performed with an incomplete adjuvant, and after another 2 weeks, analysis was performed. As a result, the PBMCs cultured with P4 and then transplanted exhibited engraftment ability equivalent to that of a control group, and specific antibody production was also maintained (Figure 8). On the other hand, the cells cultured with COR had low engraftment ability as compared with that of the control group and the P4 transplanted group, and in addition, specific antibody production was also suppressed.

Besides, in human cells engrafted into the spleen of the mouse to which P4-treated PBMCs has been transplanted, expression of CD62L that is a marker for localization of lymphocytes in the lymph node was increased as compared with that in a CTRL mouse (Figure 9, upper panels). This tendency was also found when P4 was continuously administered to a humanized NOG mouse of a GVHD onset model (Figure 9, lower panels), and it was revealed that P4 maintained CD62L expression *in vivo* for a long period of 4 weeks.

From the above results, it was revealed that COR treatment reduces the engraftment ability of PBMCs, and reduces cytotoxic activity and B cell function, but P4 maintains the number of cells in an immune system, and maintains the function thereof.

Atezolizumab, that is, an ICI, is an anti-PD-L1 antibody, and has been revealed to bind to a PD-L1 expressing tumor cell to inhibit exhaustion of a PD-1 expression T cell locally in tumor. Since PD-L1 is also expressed in activated T lymphocyte and B lymphocyte, introduction of an exhaustion signal between lymphocytes is also inhibited. Therefore, however, activation of an autoreactive T cell is promoted in many cases to cause autoimmune disease-like side effects. First, we cultured human PBMCs in the presence of TSST-1 that is a superantigen for 3 days in 5% CO₂ at 37°C, and analyzed influence of a PD-L1 antibody on PD-L1 expression in a lymphocyte by flowcytometry.

As a result, it was revealed that, in PBMCs cultured with addition of atezolizumab and simulation by TSST-1, PD-L1 expression in a T cell was reduced as compared with that in PBMCs cultured without the addition (Figure 10, second raw from left).

Besides, when P4 or COR was added to such a culture system, expression of PD-L1 was reduced along with reduction in PD-1 expression dependent on P4 concentration in a T cell, PD-L1 expression was further reduced by the addition of atezolizumab, and there was substantially no T cell expressing PD-L1 when 200 µM P4 was added. On the other hand, although a B cell minimally expresses PD-1, a B cell showed PD-L1 expression even in the presence of P4 and COR (Figure 10, lower panels). In particular, the PD-L1 expression was high at a P4 concentration of 20 µM, and this expression was reduced by the addition of atezolizumab. Based on these results, it was revealed that the function of P4 to inhibit PD-L1 expression on a B cell is low, and that the expression can be suppressed at a physiological concentration of 20 µM when atezolizumab is used in combination.

On the other hand, it was revealed that PD-1 expression in a T cell and a B cell is increased by administration of atezolizumab (Figure 11). It was revealed that this expression was also reduced when P4 is used in combination. Also in a humanized mouse *in vivo,* it was observed that the PD-L1 expression tended to be reduced and the PD-1 expression tended to be increased by the administration of atezolizumab (data not shown).

Based on these results, it was revealed that P4 reduces the expression of PD-1/PD-L1 in a T cell depending on the concentration, but has a low effect of reducing the expression of PD-L1 in a B cell, and that combined use of both of the agents effectively suppresses the expression of PD-L1 in a T cell and a B cell.

Next, a conjugate (D2125) was produced by binding the liposome-encapsulated P4 to atezolizumab. This was added to a PBMC culture system in an amount corresponding to 20 µM P4 to compare with P4/atezolizumab co-culture. As a result, a significant difference was not detected in change in the number of cells from PBMCs (Figure 12). In comparison by using PD-1 and PD-L1 expression in a T cell (positive cell percentage and MFI) as indicators, however, D2125 showed reduction in a ratio of PD-1/PD-L1 expressing cells and MFI, suppressed activation more significantly than addition of 20 µM P4, and in addition, showed T cell activation suppression ability equivalent to or more than P4 at a 10-fold concentration (Figure 13). Besides, MFI of PD-1 was slightly higher than that of 200 µM P4, but there was no significant difference, and thus, it was revealed that the MFI was efficiently reduced. Besides, the number of CD25-positive cells in CD4 T cells tended to increase (Figure 14, left panel), but IL-10 production was suppressed (data not shown). It was revealed that activation of a CD8 T cell tended to be more strongly suppressed than that of a CD4 T cell (Figure 14, right panel).

These results revealed that D2125 significantly suppresses activation of a T lymphocyte at a P4 concentration close to the physiological P4 concentration in blood of the placental villous lumen.

In conclusion, P4 suppresses activation of a T cell at a high concentration more strongly than COR, and the effect is reversible. On the other hand, it suppressed exhaustion of a T cell, and made a contribution to survival of a cytotoxic T cell and B cell function. Besides, it maintains expression of CD62L that induces localization of a lymphocyte in the lymph node. As a result, it suppresses GVHD, and maintains specific antibody production ability. Besides, it reduces expression of PD-1 and PD-L1 on a T cell, and on the other hand, does not suppress reduction in PD-L1 expression on a B cell by atezolizumab. When both of the agents are used in combination at a high concentration, PD-1/PD-L1 signals can be suppressed. Further, P4 and liposome-encapsulated P4 to which atezolizumab is bound can efficiently suppress activation of a lymphocyte at a P4 concentration in the placental lumen, that is, a high physiological concentration. In this manner, P4 and a conjugate thereof are capable of transient suppression of T cell function, and reactivation thereof with Th1/Th2 balance maintained, and therefore, are presumed to have excellent functions as an adjustor for immune system overactivation caused by ICI in autoimmune disease, transplantation immunity, and cancer treatment.

### [Example 2]

### [Material and Method]

### (1) Tumor Cell and Mouse

A breast cancer cell line MDA-MB231 used herein was one stored in Department of Molecular Life Sciences, Basic Medical Science and Molecular Medicine, Tokai University School of Medicine, and was cultured using Leibovitz L-15 medium, 15% FCS in a CO₂ free manner at 37°C. NOG mice were purchased from INVIVO Science. CMV-NOG-hIL-4-Tg mice used herein were those produced by Central Institute for Experimental Animals, and maintained in an isolator of Institute for Experimental Animals, Tokai University School of Medicine, or maintained in Central Institute for Experimental Animals. A human IL-4 concentration thereof was measured by DNA typing and ELISA to be used for transplantation.

A CMV-NOG-hIL-4-Tg mouse is a transgenic NOG mouse into which an expression vector having CMV promoter, cDNA of human IL-4, and SV40poly(A) has been introduced.

### (2) ELISA

Human IL-4 protein was quantitatively determined with Human IL-4 ELISA Set BD OptEIATM (manufactured by BD Biosciences, Catalog No.: 555194, Lot No.: 9189127). The quantitative determination of IgG antibody was performed in accordance with previously reported paper (Kametani Y., et al., Exp Hematol (2006) 34(9): 1240-1248). Wells of a microtiter plate (manufactured by Sumiron) were coated with CH401MAP peptide dissolved in carbonate buffer (pH 9.5) to cause the antigen to be adsorbed to the plate overnight at 4°C. The wells were then washed with PBS-Tween (0.05% v/v), and reacted with 3% BSA-PBS at room temperature for 2 hours. After washing the resultant with PBS-Tween 3 times, a mouse plasma was added thereto in 10-fold dilution series, followed by a reaction at room temperature for 2 hours. The resultant plate was washed 3 times, and biotin-conjugated mouse anti-human IgG mAb (manufactured by BD Pharmingen) (1:3,000) was added thereto. The resultant plate was subjected to a reaction at 37°C for 2 hours, and then washed 3 times, and streptavidin-horseradish peroxidase (1:50,000 v/v; manufactured by BD Pharmingen) was added thereto. The resultant plate was subjected to a reaction at room temperature for 1 hour, and then washed, and EIA substrate kit solution (manufactured by Bio-Rad Laboratories) was added thereto. The reaction was stopped with 10% HCl, and an absorbance at 450 nm was measured. In the quantitative determination, a calibration curve created based on standard samples having human IL-4 concentrations of 500 pg/ml, 250 pg/ml, 125 pg/ml, 62.5 pg/ml, 31.3 pg/ml, 15.6 pg/ml, and 7.8 pg/ml was used.

### (3) Preparation of Human PBMCs

30 mL of heparinized peripheral blood was collected from a healthy donor with Vacutainer ACD Tubes (manufactured by NIPRO Corporation). Immediately after that, the collected peripheral blood was layered on Ficoll-Hypaque (manufactured by SIGMA-ALDRICH), and a mononuclear cell fraction was collected by specific gravity centrifugation (500 × g, 30 minutes, 20°C). The resultant cells were washed with PBS by centrifugation at 300 × g for 5 minutes at 4°C, and then the number of the cells was counted for use.

### (4) Transplantation to PBMC-NOG-hIL-4-Tg Mouse and Analysis of Engrafted Human Cell

5 × 10⁶ MDA-MB231 was subcutaneously administered to a ventral portion of a 6- to 7-week-old NOG-hIL-4-Tg mouse (a hIL-4 concentration in blood of 100 pg/mL or more). Diameters of the tumor along the ventral and in a direction at a right angle to the ventral were measured with a caliper every 2 days from one week after the administration, and the product thereof was defined as the size of the tumor. Two weeks after the tumor transplantation, 5 × 10⁶ PBMCs were intravenously transplanted to the cancer-bearing mice and non-cancer-bearing mice as a control group. The measurement of the tumor diameters was similarly continued after the PBMC transplantation. The non-cancer bearing mice were divided into two groups, and PBS or atezolizumab (450 µg/head/dose, 3 times (every 10 days)) was administered to each group. The cancer-bearing mice were divided into three groups, and PBS, atezolizumab (450 µg/head/dose, 3 times (every 10 days)), or liposome (amount equivalent to 225 µg of atezolizumab and 40 µg of P4/dose, 6 times (every 5 days)) was administered to each group.

After 4 weeks, the mice were anesthetized to collect heparinized blood, and were euthanized. From various lymph tissues collected, cells were collected, and erythrocytes were removed therefrom with a hemolysis buffer to prepare a cell suspension. The number of cells therein was counted, and a human leukocyte fraction was subjected to flowcytometry (FCM) analysis. A tumor tissue was used for immunohistochemical staining.

### (5) Flowcytometry

For human immunocytochemistry, the same antibodies as those used in Example 1 were used. Cells were reacted with an appropriate amount of each of various, fluorescent-labeled antibodies for 15 minutes at 4°C, and washed with PBS containing 1% BSA. The resultant cells were analyzed with FACS Fortessa or Verse (manufactured by BD Bioscience). Living cells were gated, and human CD45-positive cells were further gated to obtain a human leukocyte fraction. These cells were further divided with a cell surface marker to obtain lymphocyte subsets. For data analysis, FlowJo (BD) was used.

### (6) Histochemical Staining

A tissue of the mouse was immobilized with 20% formalin (manufactured by Wako Pure Chemical Industries, Ltd.), and embedded in paraffin. The resultant paraffin block was sliced, and the resultant was subjected to deparaffinization, mounted on a slide glass, and stained with hematoxylin and eosin (HE). In immunohistochemical staining with an anti-human CD8 antibody, the slide was subjected to a heat treatment at 120°C for 20 minutes, and subjected to an endogenous peroxidase treatment through a reaction with 0.3% H₂O₂/MetOH at room temperature for 10 minutes. Thereafter, the resultant was blocked with 1% goat serum, and anti-human CD8 (manufactured by Dako) was added thereto, followed by a reaction at 25°C for 60 minutes. The resultant slide was washed 3 times with 0.01 M PBS for 5 minutes, followed by a reaction at 25°C for 60 minutes with simple stain MAX-PO (manufactured by Nichirei Biosciences Inc.). The resultant slide was washed 3 times with 0.01 M PBS for 5 minutes, and colored with a DAB solution. The resultant was washed with running water for 3 minutes, and stained with hematoxylin, followed by dehydration and penetration/encapsulation.

### (7) Statistical Processing

Statistical processing was performed with Microsoft Excel (manufactured by Microsoft). Data was indicated as mean ± SD. A significant difference test was performed by One-way ANOVA, or two-sided Student's t-test analysis.

### [Results]

In the NOG-hIL-4-Tg mouse having CMV promoter (IL-4 concentration of 100 to 500 pg/ml, CMV-NOG-hIL-4-Tg mouse), it has been found that transplantation of the human PBMCs results in a high engraftment rate of a B cell, and engraftment of CD4-positive helper T cell and CD8-positive killer T cell in a good balance, although data is not shown. Besides, in a cancer-bearing immunodeficient mouse obtained by transplanting human breast cancer cell line MDA-MB-231 and human PBMCs to a CMV-NOG-hIL-4-Tg mouse, it has been found that a ratio of a T cell in CD45-positive cells is reduced and a ratio of a B cell is increased as observed in peripheral blood mononuclear cells of an actual breast cancer patient. In other words, when a human peripheral blood mononuclear cell is transplanted to the CMV-NOG-hIL-4-Tg mouse having MDA-MB231 transplanted thereto used in the present example, an *in vivo* lymphocyte profile of a breast cancer patient can be accurately reproduced.

In the present example, atezolizumab, liposome-encapsulated P4 bound to atezolizumab, or PBS (control) was administered to a cancer-bearing immunodeficient mouse obtained by transplanting human breast cancer cell line MDA-MB-231 and human PBMCs to a CMV-NOG-hIL-4-Tg mouse, and CD19-positive B cell, CD3-positive T cell, CD4-positive helper T cell, and CD8-positive killer T cell engrafted into spleen cells were measured. Besides, in a cancer-bearing immunodeficient mouse to which atezolizumab, liposome-encapsulated P4 bound to atezolizumab, or PBS (control) had been administered, a tumor tissue was observed by histochemical staining to observe a degree of infiltration of lymphocytes.

Results of measurement of CD19-positive B cell, CD3-positive T cell, CD4-positive helper T cell, and CD8-positive killer T cell engrafted into the spleen cells by flowcytometry are illustrated in Figure 15, and results of observation of the tumor tissues by histochemical staining are illustrated in Figure 16. As illustrated in Figure 15, when atezolizumab was administered to the CMV-NOG-hIL-4-Tg mouse having the breast cancer cell line MDA-MB231 transplanted thereto, the engraftment of a B cell was reduced, and the ratio of a T cell was increased, and particularly, the ratio of the CD8-positive killer T cell was increased. Besides, when the liposome-encapsulated P4 bound to atezolizumab was administered, the engraftment of a B cell was further reduced, and the ratio of a T cell was further increased, and particularly, the ratio of the CD8-positive killer T cell was also further increased as compared with the case where atezolizumab alone was administered. Besides, as the results of the histochemical staining of the tumor tissues, lymphocyte infiltration into a tumor mass was not observed in a control PBS administration group, but human T cell infiltration into a tumor mass was obviously observed in the atezolizumab administration group, as illustrated in Figure 16. Besides, when the liposome-encapsulated P4 bound to atezolizumab was administered, regression of the tumor mass was caused more remarkably, and in addition, the infiltration of the human killer T cell into the tumor mass was observed at an equivalent or higher level, as compared with the case where atezolizumab alone was administered. Although data is not shown, the CD4-positive helper T cell also infiltrated into the tumor mass, and when the liposome-encapsulated P4 bound to atezolizumab was administered, the infiltration of the CD4-positive helper T cell was observed more remarkably as compared with the case where atezolizumab alone was administered.

Besides, results of measuring a tumor diameter when administering atezolizumab, liposome-encapsulated P4 bound to atezolizumab, or PBS (control) to the CMV-NOG-hIL-4-Tg mouse having the breast cancer cell line MDA-MB231 transplanted thereto are illustrated in Figure 17. As illustrated in Figure 17, the transplanted breast cancer increased over time as seen in a cancer-bearing immunodeficient mouse to which PBS was administered. On the contrary, in a cancer-bearing immunodeficient mouse to which atezolizumab or liposome-encapsulated P4 bound to atezolizumab was administered, the increase of the tumor was suppressed as compared with that in the PBS administration group.

It was revealed, based on these results, that liposome-encapsulated P4 bound to atezolizumab exhibits a greatly excellent anti-tumor effect as compared with atezolizumab alone, that is an immune checkpoint inhibitor for a human breast cancer patient.

All publications, patents, and patent applications cited herein are incorporated herein by reference.

## Claims

1. An agent for combination use comprising, as an active ingredient, a T cell and/or B cell activity regulator comprising progesterone or a derivative thereof as an active ingredient, for use in combination treatment with an anti-human PD-L1 monoclonal antibody.

2. The agent for combination use according to claim 1, wherein the T cell and/or B cell activity regulator reversibly acts on a T cell and/or a B cell.

3. The agent for combination use according to claim 1 or 2, wherein the T cell and/or B cell activity regulator maintains expression of CD62L to localize a T cell in a lymph node.

4. The agent for combination use according to any one of claims 1 to 3, wherein the T cell and/or B cell activity regulator reduces expression of PD-1 in a T cell and/or a B cell.

5. The agent for combination use according to any one of claims 1 to 4, wherein the agent for combination use suppresses expression of PD-L1 in a T cell and/or a B cell.

6. The agent for combination use according to any one of claims 1 to 5, wherein the agent suppresses expression of PD-1 and PD-L1 in a T cell and/or a B cell.

7. The agent for combination use according to any one of claims 1 to 6, wherein the anti-human PD-L1 monoclonal antibody is a humanized anti-human PD-L1 monoclonal antibody.

8. The agent for combination use according to claim 7, wherein the humanized anti-human PD-L1 monoclonal antibody is atezolizumab.

9. A pharmaceutical composition comprising, as active ingredients, a T cell and/or B cell activity regulator comprising progesterone or a derivative thereof as an active ingredient, and an anti-human PD-L1 monoclonal antibody.

10. The pharmaceutical composition according to claim 9, wherein the T cell and/or B cell activity regulator reversibly acts on a T cell and/or a B cell.

11. The pharmaceutical composition according to claim 9 or 10, wherein the T cell and/or B cell activity regulator maintains expression of CD62L to localize a T cell in a lymph node.

12. The pharmaceutical composition according to any one of claims 9 to 11, wherein the T cell and/or B cell activity regulator reduces expression of PD-1 in a T cell and/or a B cell.

13. The pharmaceutical composition according to any one of claims 9 to 12, wherein the pharmaceutical composition suppresses expression of PD-L1 in a T cell or a B cell.

14. The pharmaceutical composition according to any one of claims 9 to 13, wherein the pharmaceutical composition suppresses expression of PD-1 and PD-L1 in a T cell or a B cell.

15. The pharmaceutical composition according to any one of claims 9 to 14, wherein the anti-human PD-L1 monoclonal antibody is a humanized anti-human PD-L1 monoclonal antibody.

16. The pharmaceutical composition according to claim 15, wherein the humanized anti-human PD-L1 monoclonal antibody is atezolizumab.

17. An immunoliposome comprising a liposome comprising a T cell and/or B cell activity regulator comprising progesterone or a derivative thereof as an active ingredient, wherein an anti-human PD-L1 monoclonal antibody is bound to a membrane of the liposome.

18. The immunoliposome according to claim 17, wherein the T cell and/or B cell activity regulator reversibly acts on a T cell and/or a B cell.

19. The immunoliposome according to claim 17 or 18, wherein the T cell and/or B cell activity regulator maintains expression of CD62L to localize a T cell in a lymph node.

20. The immunoliposome according to any one of claims 17 to 19, wherein the T cell and/or B cell activity regulator reduces expression of PD-1 in a T cell and/or a B cell.

21. The immunoliposome according to any one of claims 17 to 20, wherein the immunoliposome suppresses expression of PD-L1 in a T cell and/or a B cell.

22. The immunoliposome according to any one of claims 17 to 21, wherein the immunoliposome suppresses expression of PD-1 and PD-L1 in a T cell and/or a B cell.

23. The immunoliposome according to any one of claims 17 to 22, wherein the anti-human PD-L1 monoclonal antibody is a humanized anti-human PD-L1 monoclonal antibody.

24. The immunoliposome according to claim 23, wherein the humanized anti-human PD-L1 monoclonal antibody is atezolizumab.

25. A pharmaceutical composition comprising the immunoliposome according to any one of claims 17 to 24.
